# FASCICULE DE BREVET EUROPEEN

(11) **EP 2 654 938 B1**
(45) Date de publication et mention de la délivrance du brevet: **08.10.2014**
(21) Numéro de dépôt: 11817393.9
(22) Date de dépôt: 19.12.2011
(51) Int. Cl.: B01F 13/00, B01F 13/10, B01F 15/02, A61M 5/14, A61M 5/168, A61M 5/19, A61M 5/20, A61M 5/162, B01F 5/06, A61J 3/00, A61M 39/22

(54) **DISPOSITIF POUR MÉLANGER AU MOINS DEUX CONSTITUANTS**
VORRICHTUNG ZUM MISCHEN VON MINDESTENS ZWEI BESTANDTEILEN
DEVICE FOR MIXING AT LEAST TWO CONSTITUENTS

(30) Priorité: 24.12.2010 FR 1061242
(43) Date de publication de la demande: 30.10.2013
(73) Titulaire: Eveon, 38330 Montbonnot Saint Martin (FR)
(72) Inventeur: DEHAN, Christophe, F-38330 Saint Ismier (FR); LAMY, Didier, F-74330 Poisy (FR)
(74) Mandataire: Prugneau, Philippe
(86) Numéro de dépôt international: PCT/FR2011/053054
(87) Numéro de publication internationale: WO 2012/085428

(56) Documents cités:
- EP-A2- 0 013 334
- DE-A1- 4 440 987
- US-A1- 2002 112 530
- US-A1- 2005 284 886

## Description

### Domaine technique

L'invention concerne un dispositif pour mélanger au moins deux constituants provenant d'au moins un premier réservoir et un second réservoir distinct du premier réservoir, dispositif pour mélanger comprenant au moins un premier orifice apte à être raccordé au premier réservoir, et au moins un second orifice distinct du premier orifice et apte à être raccordé au second réservoir et une pompe motorisée. Ce dispositif pour mélanger permettant d'obtenir un mélange homogène.

L'invention concerne également un dispositif d'injection miniaturisé à usage médical comprenant un tel dispositif pour mélanger.

### Technique antérieure

De nombreux mélanges de constituants, par exemple à vocation thérapeutique dans le domaine médical ou animal ou à d'autres fins dans le domaine cosmétique, sont peu stables dans leur état prêt à être administré. Aussi, les différents constituants de ces mélanges sont conservés à l'état séparé dans différents flacons et mélangés juste avant administration par l'utilisateur. Par exemple, on présente souvent un premier constituant sous forme poudre solide ou de lyophilisé déshydraté à reconstituer avec un second constituant de type solvant, ou encore sous une forme concentrée avec une forte viscosité à diluer par un solvant, ce qui nécessite un protocole de reconstitution ou mélange adapté pour obtenir une solution homogène.

Par administration, on entend tout acte permettant de faire absorber un constituant à un utilisateur, que ce soit par une injection, une perfusion, par voie orale ou cutanée, ou par tout autre moyen.

Classiquement, entre cinq et dix étapes manuelles sont nécessaires pour obtenir le mélange de ces constituants, mettant en jeu des seringues et des flacons, à savoir : prélever un constituant dans l'un des flacons avec une seringue, le transférer dans un autre flacon contenant le second constituant, en évitant les effets d'émulsion ou d'agglomération, brasser le mélange afin d'obtenir une solution homogène des deux constituants, prélever la solution finale avec une seringue, administrer au patient.

On connaît, par exemple du document de brevet FR-2 708 204, un dispositif tel que décrit plus haut comprenant un corps de seringue équipé d'un piston pré-rempli d'un premier constituant liquide. Le dispositif est muni de moyens de fixation du corps de seringue sur un flacon médical standard rempli du second constituant. Pour réaliser le mélange des deux constituants, le corps de seringue est fixé sur le flacon et le constituant liquide est injecté manuellement dans le flacon à l'aide du piston. Un inconvénient de ce dispositif est qu'il requiert un pré-remplissage de la seringue, ce qui est moins pratique et plus cher que l'utilisation de flacons standards. Un dispositif analogue est décrit dans DE 4440987 A1.

De manière générale, l'ensemble des étapes d'obtention du mélange et de brassage nécessite l'utilisation de trois à huit consommables stériles (seringues, aiguilles...) afin de respecter des conditions d'hygiène convenables. De plus, l'utilisation répétée de seringues avec aiguille multiplie les risques de blessures avec l'aiguille. Enfin, ces étapes sont aussi assorties de temps d'attente. C'est pourquoi l'administration de ces mélanges de constituants requiert l'aide d'un professionnel médical ou une formation de l'utilisateur.

Or, actuellement, environ 50% des nouvelles molécules thérapeutiques développées sont d'origine biologique, conservables principalement sous une forme poudre solide ou lyophilisée à reconstituer juste avant administration.

Il est de plus primordial d'assurer la sécurité des personnes réalisant ces mélange, en particulier lorsque le mélange met en jeu des substances cytotoxiques. Pour assurer la sécurité, certaines installations d'aspiration d'air sont parfois utilisées. Ces installations représentent un coût supplémentaire non négligeable et ne peuvent être utilisées que dans des lieux spécifiques dédiés ce qui ne permet pas de répondre à la majorité des cas d'utilisation. Aussi, il est nécessaire de proposer des dispositifs de mélange de type « fermé » permettant un mélange sans risque de contamination par le biais de liquide, poudre ou vapeur et permettant de garantir un bon niveau d'asepsie.

Il existe donc un réel besoin pour des dispositifs médicaux permettant d'effectuer des mélanges de constituants de façon simple, sûre et indépendante de l'utilisateur, et optionnellement d'administrer ces mélanges.

### Exposé de l'invention

Le but de l'invention est de répondre à ce besoin tout en palliant les inconvénients des dispositifs existants en proposant un dispositif pour mélanger permettant le mélange de deux constituants, qui est de manipulation très simple et peut être utilisé par n'importe quel utilisateur non formé médicalement et optionnellement intégré à un dispositif d'injection miniaturisé à usage médical. Le but de l'invention est également de proposer un dispositif pour mélanger permettant d'obtenir un mélange homogène. Les avantages sont multiples :
- limitation du risque de blessure par les objets piquants habituellement utilisés (aiguilles),
- limitation du temps de manipulation (automatisation, reconstitution sans intervention de l'opérateur),
- faible exposition ou absence d'exposition aux molécules actives manipulées qui peuvent être dangereuse pour un opérateur qui n'est pas le patient.

A cet effet, l'invention a pour objet un dispositif pour mélanger au moins deux constituants provenant d'au moins un premier réservoir et un second réservoir distinct du premier réservoir, le dispositif pour mélanger comprenant au moins un premier orifice apte à être raccordé au premier réservoir, et au moins un second, orifice distinct du premier orifice et apte à être raccordé au second réservoir et une pompe motorisée, caractérisé en ce qu'il comprend un sélecteur fluidique raccordé à la pompe et comprenant un réseau de canaux, le sélecteur fluidique étant pourvu d'au moins :
- une portion fixe comprenant au moins une partie du réseau de canaux débouchant au moins par le premier orifice et par le second orifice,
- une portion mobile comprenant au moins une autre partie du réseau de canaux et mobile entre plusieurs positions comprenant au moins :
- une première position dans laquelle le réseau de canaux met en communication l'un au moins des premier orifice et second orifice et la pompe pour permettre, à l'aide de la pompe, un transfert d'au moins un constituant du premier orifice vers le second orifice par l'intermédiaire de la pompe,
- une seconde position dans laquelle le réseau de canaux met en communication le second orifice et la pompe pour permettre à l'aide de la pompe, un brassage par circulation dans le réseau de canaux du mélange obtenu.

Par pompe « motorisée », on entend une pompe pourvue de moyens servant à transformer une énergie quelconque, comme par exemple électrique, en énergie mécanique. Selon cette définition, une pompe à membrane notamment fait partie des pompes motorisées.

Avec le dispositif pour mélanger selon l'invention, le mélange (ou reconstitution) et le brassage des constituants est facilité : après fixation de deux réservoirs contenant respectivement les deux constituants sur le dispositif pour mélanger, le sélecteur fluidique est réglé pour relier les orifices menant aux réservoirs entre eux, puis la pompe motorisée est actionnée pour faire circuler les constituants d'un réservoir à l'autre de sorte à réaliser le mélange, puis brasser le mélange dans un réservoir.

Ces opérations très simples peuvent être réalisées directement par l'utilisateur, même non formé aux techniques médicales. De plus, le mélange et le brassage étant automatisés, les risques d'erreurs sont réduits. En outre, des flacons standards peuvent être directement utilisés comme réservoir et le nombre de consommables stériles nécessaires est réduit. Enfin, le mélange circulant dans le réseau de canaux avant d'être utilisé, on est assuré de la précision du dosage entre les différents constituants.

Un dispositif pour mélanger selon l'invention peut présenter avantageusement les particularités suivantes:
- la pompe est au moins apte à être couplée à un réservoir de pompe ;
- le dispositif pour mélanger peut comporter deux seconds orifices aptes à être couplés au second réservoir de sorte que dans la seconde position, le brassage est obtenu par circulation du mélange entre les seconds orifices par la pompe ;
- le réseau de canaux du sélecteur fluidique comporte au moins un canal de sortie pour évacuer le mélange du dispositif, la portion mobile du sélecteur fluidique pouvant occuper une troisième position dans laquelle ledit réseau de canaux met en communication la pompe ou le second orifice au canal pour évacuer ledit mélange du dispositif pour mélanger ;
- le dispositif pour mélanger peut comporter au moins un troisième orifice apte à être raccordé à un troisième réservoir distinct des premier et second réservoirs, la portion mobile du sélecteur fluidique étant apte à être dans une troisième position dans laquelle le réseau de canaux met en communication le second orifice et le troisième orifice pour permettre le transfert du mélange du second orifice vers le troisième orifice ;
- l'un au moins des premier orifice, second orifice et troisième orifice est formé individuellement sur un embout et, lorsque le dispositif pour mélanger comporte deux seconds orifices, les embouts portant ces derniers présentent des longueurs différentes, permettant d'améliorer la circulation fluidique dans le second réservoir ;
- l'un au moins des premier orifice, second orifice et troisième orifice est formé individuellement sur un embout et, lorsque le dispositif pour mélanger comporte deux seconds orifices présentant des sections terminales de dimensions intérieures différentes entre elles, permettant d'améliorer la circulation fluidique dans le second réservoir ;
- le sélecteur fluidique est agencé pour être couplé à un entraînement motorisé apte à le déplacer entre les première, seconde, troisième positions ;
- le dispositif pour mélanger peut comporter un premier ensemble jetable comportant le sélecteur fluidique, et un second ensemble réutilisable comportant l'entraînement motorisé de la pompe et du sélecteur fluidique, le premier ensemble jetable et le second ensemble réutilisable étant agencés pour être connectables et détachables respectivement l'un de l'autre ;
- le premier ensemble jetable comprend au moins le premier réservoir contenant un solvant et le second réservoir contenant une poudre solide ou un lyophilisé ;
- la portion mobile du sélecteur fluidique est agencée pour être mobile en rotation par à la portion fixe ;
- la portion mobile du sélecteur fluidique est agencée pour être mobile en translation par rapport à la portion fixe ;

L'invention s'étend à un dispositif d'injection miniaturisé à usage médical caractérisé en ce qu'il comprend un dispositif pour mélanger tel que décrit ci-dessus et une aiguille raccordée audit sélecteur fluidique.

Dans le dispositif d'injection miniaturisé à usage médical selon l'invention, l'aiguille peut avantageusement être montée mobile dans une enceinte dudit dispositif d'injection.

### Description sommaire des dessins

La présente invention sera mieux comprise et d'autres avantages apparaîtront à la lecture de la description détaillée de quelques modes de réalisation pris à titre d'exemples nullement limitatifs et illustrés par les dessins annexés dans lesquels :
- la figure 1 est une vue en perspective schématique du dispositif pour mélanger selon un premier mode de réalisation de l'invention ;
- la figure 2 est une vue en perspective et en éclaté du dispositif pour mélanger de la figure 1 ;
- la figure 3 est une vue en coupe selon un plan vertical passant par l'axe III-III de la figure 1 du dispositif pour mélanger de la figure 1 ;
- la figure 4 représente une partie du dispositif pour mélanger de la figure 1 ;
- la figure 5 représente une autre partie du dispositif pour mélanger de la figure 1 ;
- la figure 6 représente une autre partie encore du dispositif pour mélanger de la figure 1 ;
- la figure 7 est un organigramme représentant des étapes de l'utilisation du dispositif pour mélanger selon l'invention ;
- la figure 8 est une vue en perspective en éclaté du dispositif pour mélanger selon un second mode de réalisation de l'invention ;
- la figure 9 est une vue agrandie d'une partie du dispositif pour mélanger de la figure 8 ;
- la figure 10 est une vue en perspective en éclaté du dispositif pour mélanger selon un troisième mode de réalisation de l'invention ;
- la figure 11 est une vue en coupe selon un plan vertical passant par l'axe XI-XI de la figure 10 du dispositif pour mélanger de la figure 10 ;
- la figure 12 est une vue agrandie en coupe selon le plan vertical passant par l'axe XI-XI de la figure 10 d'une partie du dispositif pour mélanger de la figure 10 ;
- la figure 13 est une vue agrandie en perspective de la partie du dispositif pour mélanger de la figure 12 ;
- la figure 14 est une vue en perspective en éclaté du dispositif pour mélanger selon un quatrième mode de réalisation de l'invention ;
- la figure 15 est une vue en coupe selon le plan vertical passant par l'axe XV-XV de la figure 14 du dispositif pour mélanger de la figure 14 ;
- la figure 16 est une vue en coupe selon le plan vertical passant par l'axe XVI-XVI de la figure 14 du dispositif pour mélanger de la figure 14 ;
- la figure 17 est une vue agrandie en perspective d'une partie du dispositif pour mélanger de la figure 14 ;
- la figure 18 est une vue schématique d'un dispositif d'injection selon l'invention comprenant le dispositif pour mélanger de la figure 1.

### Description des modes de réalisation

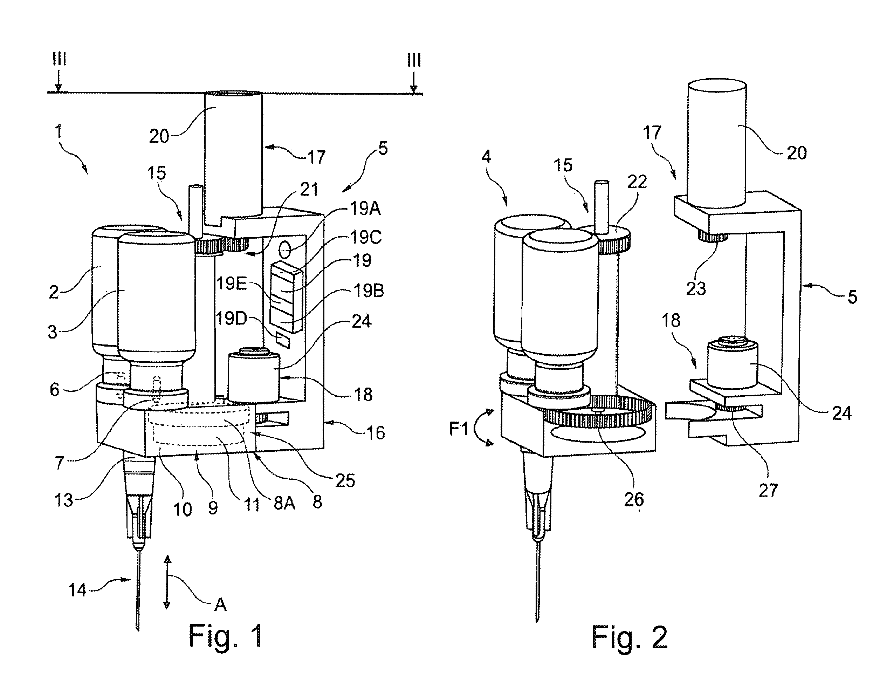

Sur la figure 1, on a représenté un dispositif 1 pour mélanger miniaturisé à usage médical selon l'invention permettant le mélange de deux constituants respectivement contenus dans un premier réservoir 3 et un second réservoir 2. De préférence, on utilise des flacons médicaux standards, appelés couramment « vials » ou « cartouches » ou « poches », comme premier et second réservoirs 3, 2.

Le dispositif 1 pour mélanger comprend sur un socle 8 d'une part deux embouts pourvus respectivement d'un premier orifice 7 et d'un second orifice 6 aptes à recevoir chacun un des premier réservoir 3 et second réservoir 2 de manière étanche de sorte à conserver la stérilité originelle des premier et second réservoir 3, 2, et d'autre part un canal de sortie 13 pour évacuer le mélange, ici par exemple par une aiguille 14 d'injection.

De préférence, le dispositif 1 pour mélanger est avantageusement inséré dans un dispositif d'injection miniaturisé à usage médical comprenant une enceinte (non représentée) et l'aiguille 14 est montée mobile dans cette enceinte selon une direction axiale indiquée par la flèche A sur la figure 1, grâce à des moyens de déplacement 14A adaptés pour déplacer l'aiguille 14 dans une position saillante hors de l'enceinte afin de réaliser une injection du mélange. De préférence, les moyens de déplacement 14A sont motorisés.

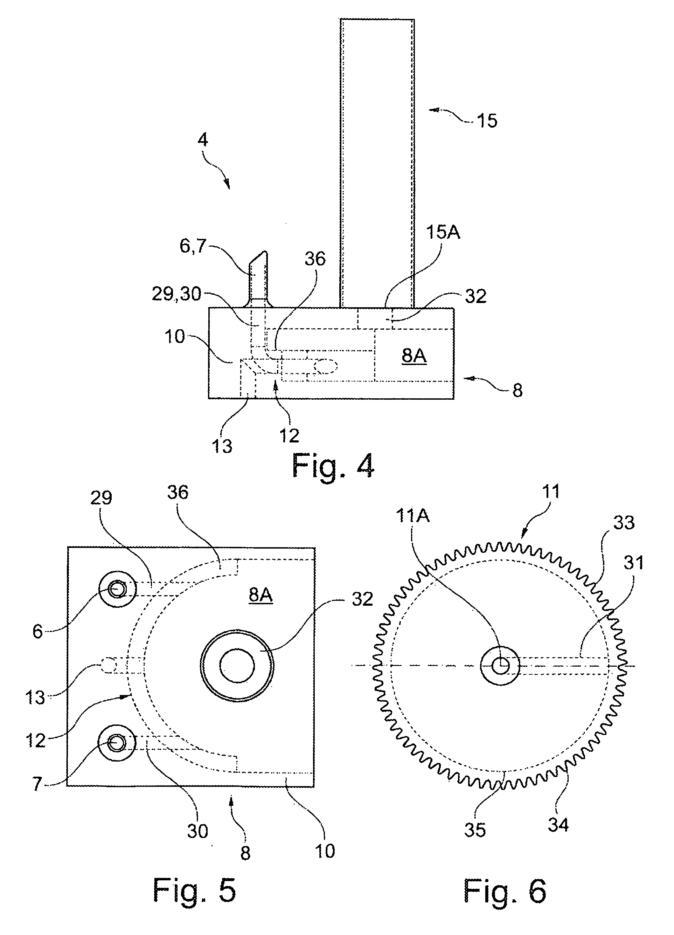

Le socle 8 forme une portion fixe 10 d'un sélecteur fluidique 9 rotatif et est creusé d'un évidement 8A sensiblement demi-circulaire (détaillé sur la figure 5) de sorte à recueillir une portion mobile 11 sensiblement circulaire du sélecteur fluidique 9. Comme on le verra plus particulièrement en référence aux figures 4 à 6, le sélecteur fluidique 9 comprend dans ses deux portions fixe et mobile 10, 11 un réseau de canaux 12 dont les parties de la portion fixe 10 et de la portion mobile 11 peuvent être mises en coïncidence de sorte à relier sélectivement les embouts pourvus des premier et second orifices 7, 6 et le canal de sortie 13. De préférence, le sélecteur fluidique 9 peut être réalisé avec des pièces de plasturgies simples, d'une manière connue en soi.

On notera que les embouts pourvus des premier et second orifices 7, 6 sont de préférence équipés d'un moyen de perçage (non représenté) d'un bouchon des premier et second réservoirs 3, 2, et d'une garniture d'étanchéité pour maintenir la liaison étanche entre les premier et second réservoirs 3, 2 et le réseau de canaux 12 du sélecteur fluidique 9.

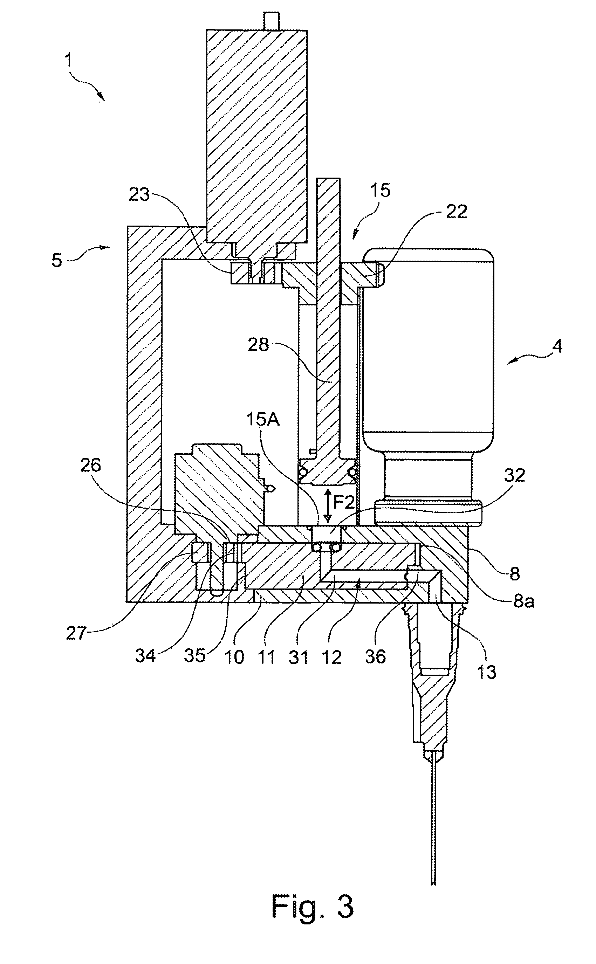

Le dispositif 1 pour mélanger comprend aussi, montée sur le socle 8, une pompe 15 motorisée, ici de type pompe à piston bi-directionnelle à un port 15A unique visible sur la figure 3, adaptée pour faire circuler les constituants dans le réseau de canaux 12 du sélecteur fluidique 9, entre les embouts pourvus des premier et second orifices 7, 6 et le canal de sortie 13.

Comme on peut aussi le voir sur la figure 1, le dispositif 1 pour mélanger comprend en outre sur un châssis 16 un premier entraînement 17 motorisé de la pompe 15 et un second entraînement 18 motorisé du sélecteur fluidique 9 qui sont commandés par une unité centrale de commande et de contrôle 19 montée sur le châssis 16, ou en variante sur l'enceinte du dispositif 1 pour mélanger.

Plus particulièrement, le premier entraînement motorisé 17 de la pompe 15 comprend un premier moteur 20 et un premier ensemble de transmission 21 relié à la pompe 15, qui se présente ici sous la forme d'un premier couple d'engrenages 22, 23 qui s'engrènent l'un dans l'autre, l'un 22 relié à la pompe 15 et l'autre 23 relié au premier moteur 20, comme on peut mieux le voir sur la figure 2.

De manière similaire, le second entraînement motorisé 18 du sélecteur fluidique 9 comprend un second moteur 24 et un second ensemble de transmission 25 relié au sélecteur fluidique 9, qui se présente ici sous la forme d'un second couple d'engrenages 26, 27 qui s'engrènent l'un dans l'autre, l'un 26 fixé sur la portion mobile 11 du sélecteur fluidique 9 et l'autre 27 relié au second moteur 24. Ainsi, quand l'entrainement motorisé 18 entraîne en rotation l'engrenage 27, l'engrenage 26 est entraîné selon la double-flèche F1 indiquée sur la figure 2 pour mettre en coïncidence les différents canaux du réseau de canaux 12 sélectivement, comme cela sera décrit plus loin.

Par unité centrale de commande et de contrôle 19, on entend tous les composants nécessaires au fonctionnement automatique du dispositif 1 pour mélanger, notamment un ou plusieurs microcontrôleurs des moteurs et les interconnexions internes nécessaires. Par ailleurs, le dispositif 1 pour mélanger comprend sur le châssis 16, et/ou sur l'enceinte du dispositif 1 pour mélanger, une interface utilisateur 19A avec des boutons de commande et/ou un affichage, ainsi qu'une alimentation 19B.

Avantageusement, le dispositif 1 pour mélanger peut aussi comprendre sur le châssis 16, et/ou sur l'enceinte du dispositif 1, un ou plusieurs capteurs 19D, par exemple un capteur de présence des premier et second réservoirs 3, 2, de fluide, du patient ou de l'aiguille 14 ou encore un détecteur de l'intégrité du dispositif 1 pour mélanger, ainsi qu'une base de données 19C interne accessible par l'unité centrale de commande et de contrôle 19 et/ou un émetteur-récepteur radiofréquence 19E pour l'échange de données avec un ordinateur distant (non représenté).

On pourra aussi avantageusement prévoir, dans le dispositif 1 pour mélanger, un dispositif de verrouillage (non représenté) des premier et second réservoirs 3, 2 en place sur les embouts pourvus des premier et second orifices 7, 6 pour éviter tout risque d'échange ou de contamination des premier et second réservoirs 3, 2 pendant ou après l'utilisation du dispositif 1 pour mélanger.

Avantageusement, le dispositif 1 pour mélanger selon l'invention peut être conformé en deux ensembles 4, 5 distincts s'emboîtant l'un dans l'autre, comme cela apparaît clairement sur la figure 2. Un premier ensemble jetable 4 comprenant toutes les parties du dispositif 1 pour mélanger qui sont en contact avec les constituants à mélanger est conçu pour être stérilisé et à usage unique. Un second ensemble réutilisable 5 comprenant le reste du dispositif 1 pour mélanger est réutilisable. Ainsi, après utilisation du dispositif 1 pour mélanger, le premier ensemble jetable 4 est souillé et peut être jeté, et le second ensemble réutilisable 5 peut être réutilisé avec un autre premier ensemble jetable 4 stérile pour préparer un nouveau mélange.

Ainsi, le second ensemble réutilisable 5 du dispositif 1 pour mélanger comprend essentiellement le châssis 16 avec les deux entraînements motorisés 17, 18 et l'unité centrale de commande et de contrôle 19, alors que le premier ensemble jetable 4 comprend uniquement les parties fluidiques du dispositif 1 pour mélanger, à savoir notamment la pompe 15 et le sélecteur fluidique 9 montés sur le socle 8.

On comprendra que le premier ensemble jetable 4 inclut l'engrenage 22 relié à la pompe 15 du premier entraînement motorisé 17 et l'engrenage 26 fixé sur la portion mobile 11 du second entraînement motorisé 18, alors que le second ensemble réutilisable 5 comprend l'engrenage 23 relié au premier entraînement motorisé 17 et l'engrenage 27 relié au second entraînement motorisé 18.

Cet agencement en un premier et second ensemble jetable 4 et réutilisable 5 du dispositif 1 pour mélanger selon l'invention permet d'une part de réduire la partie fluidique à stériliser (premier ensemble jetable 4) et d'autre part de recycler la partie commande et entraînement (second ensemble réutilisable 5).

Sur la figure 3, on a représenté le dispositif 1 pour mélanger en coupe pour mieux faire apparaître les éléments mobiles du dispositif 1 pour mélanger. Comme on peut le voir, la pompe 15 comprend un piston 28 qui est équipé ici d'une vis sans fin coopérant avec l'engrenage 22 de la pompe 15. Ainsi, lorsque l'entrainement motorisé 17 entraîne en rotation les engrenages 22, 23 du premier couple d'engrenages, le piston 28 se déplace longitudinalement dans la pompe 15 dans un sens ou l'autre selon la double-flèche F2 indiquée sur la figure 3 pour faire circuler les constituants dans le dispositif 1.

Les figures 4 et 5 représentent schématiquement le premier ensemble jetable 4 du dispositif 1 pour mélanger, sans la portion mobile 11 du sélecteur fluidique 9, pour mieux montrer le réseau de canaux 12 de circulation des constituants dans le dispositif 1 pour mélanger. On voit que le socle 8 est creusé de deux premiers canaux 29, 30 reliés respectivement directement aux embouts pourvus des premier et second orifices 7, 6 et débouchant dans l'évidement 8A du socle 8 qui est relié au canal de sortie 13. Par ailleurs, le socle 8 est creusé d'un troisième canal 32 s'ouvrant d'un côté sur le port 15A de la pompe 15 et de l'autre côté sur l'évidement 8A.

En outre, la portion mobile 11, ici circulaire, du sélecteur fluidique 9 est bordée sur toute sa périphérie de dents 33 formant l'engrenage 26 du second couple d'engrenages sur une partie de longueur 34 de la portion mobile 11, comme on peut le voir sur la figure 6. Sur une autre partie de longueur 35, la portion mobile 11 est lisse et légèrement en retrait par rapport aux dents 33, ce qui donne à la portion mobile 11 un profil en T, vu en section axiale de la portion mobile 11 sur la figure 3. L'évidement 8A du socle 8 a une forme complémentaire à la portion mobile 11 avec un rebord 36 semi-circulaire mieux visible sur la figure 5, sur lequel repose la partie de longueur 34 dentée de la portion mobile 11. En variante, les dents 33 de l'engrenage 26 s'étendent sur une partie seulement de la périphérie de la portion mobile11 de sorte à réaliser une butée et limiter la rotation de la portion mobile 11 dans la portion fixe 10.

Comme on peut mieux le voir sur les figures 3 et 6, la portion mobile 11 est creusée d'un canal 31 en forme de L qui passe par le centre 11A de la portion mobile 11 et qui débouche latéralement au niveau de la partie de longueur 35 lisse de la portion mobile 11.

Ainsi, lorsque la portion mobile 11 est en position dans l'évidement 8A du socle 8, le canal 31 débouche au centre dans le troisième canal 32 menant à la pompe 15. La portion mobile 11 peut alors occuper trois positions angulaires différentes pour relier sélectivement et de manière étanche la pompe 15 avec le canal de sortie 13 ou chacun des embouts pourvus des premier et second orifices 7, 6, le canal 31 débouchant latéralement dans l'un ou l'autre du canal de sortie 13 ou des premiers canaux 29, 30 menant aux embouts pourvus des premier et second orifices 7, 6. Cet agencement circulaire du sélecteur fluidique 9 permet de bien répartir la pression du fluide dans le réseau de canaux 12 et d'obtenir une bonne étanchéité du sélecteur fluidique 9, tout en réduisant le nombre de pièces le composant.

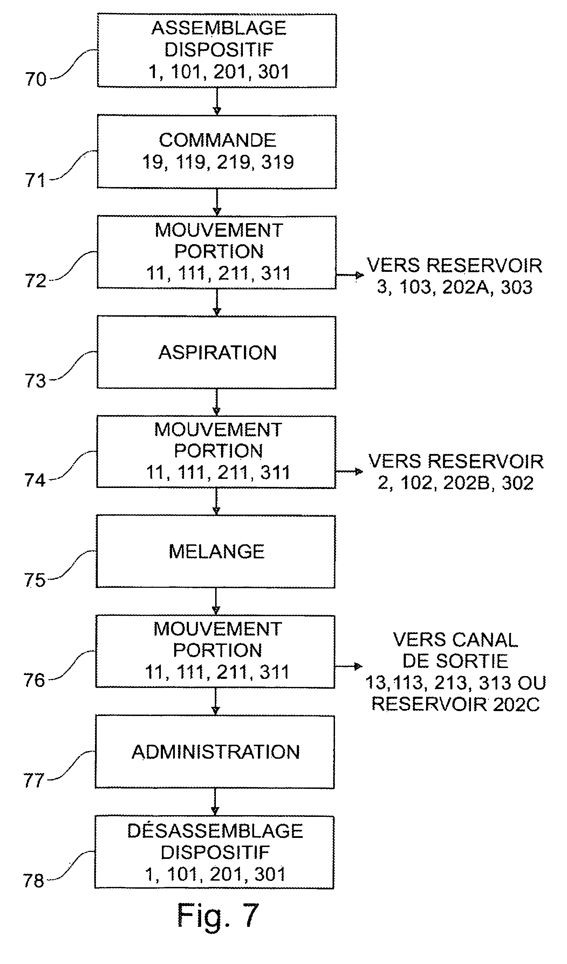

Pour utiliser le dispositif 1 pour mélanger selon l'invention et procéder au mélange et au brassage des deux constituants contenus dans les premier et second réservoirs 2, 3, l'utilisateur commence par introduire à l'étape 70 de la figure 7 un premier et un second réservoir 3, 2 sur un des embouts pourvus respectivement des premier et second orifices 7, 6, avec par exemple poudre solide ou un produit lyophilisé dans le second réservoir 2 et un solvant dans le premier réservoir 3, et assemble le dispositif 1 pour mélanger en emboîtant l'un dans l'autre les premier et second ensembles jetable 4 et réutilisable 5.

Puis, il actionne à l'étape 71 l'unité centrale 19 pour déclencher des étapes 72 à 76 du mélange qui sont avantageusement pré-programmées dans l'unité centrale de commande et de contrôle 19. Avantageusement, à cette étape 72, l'unité centrale de commande et de contrôle 19 accède à la base de données interne 19C ou à une base de données externe via l'émetteur-récepteur 19E de sorte à déterminer, sur la base d'informations données par l'utilisateur via l'interface utilisateur 19A ou fournies par les capteurs 19D, le protocole du mélange, par exemple la dose de solvant à utiliser, et le cas échéant le protocole de l'injection, par exemple la profondeur de pénétration de l'aiguille 14 dans la peau, la vitesse d'injection, etc.

A l'étape 72, l'unité centrale de commande et de contrôle 19 commande la rotation de la portion mobile 11 du sélecteur fluidique 9 de sorte à mettre en coïncidence le canal 31 de la portion mobile 11 avec le canal 30 menant à l'embout pourvu du premier orifice 7 du premier réservoir 3 de solvant.

A l'étape 73 d'aspiration, l'unité centrale de commande et de contrôle 19 actionne alors l'entrainement 17 de la pompe 15 ce qui entraine le piston 28, de sorte à aspirer dans la pompe 15 la dose de solvant désirée.

Puis, une fois la quantité de solvant nécessaire aspiré, à l'étape 74, l'unité centrale de commande et de contrôle 19 commande de nouveau la rotation de la portion mobile 11 du sélecteur fluidique 9, de sorte à mettre en coïncidence le canal 31 de la portion mobile 11 avec maintenant le canal 29 menant à l'embout pourvu du second orifice 6 du second réservoir 2 de poudre solide ou de produit lyophilisé.

A l'étape 75 de brassage, l'unité centrale de commande et de contrôle 19 actionne l'entrainement 17 de la pompe 15 de sorte à injecter le solvant, contenu dans la pompe 15, dans le second réservoir 2. Selon les constituants en présence, le protocole du mélange peut exiger un brassage du mélange pour obtenir un mélange homogène. Dans ce cas, le piston 28 de la pompe 15 peut être actionné dans les deux sens de la flèche F2 indiquée sur la figure 3 pour aspirer et refouler plusieurs fois le mélange depuis/vers le second réservoir 2. Ce brassage permet également de s'assurer, que seul le mélange homogène est présent dans le dispositif 1 pour mélanger et en particulier qu'il ne reste aucune trace de solvant pur qui risquerait d'être injecté tel quel. La précision du dosage de constituant actif du mélange en est améliorée. Lorsque le mélange est homogène selon le protocole, le mélange est aspiré dans la pompe 15. A ce moment, en vue de l'administration du mélange à un patient, le mélange peut être soit stocké dans un des premier et second réservoirs 3, 2, ou dans la pompe 15 qui constitue alors un réservoir de pompe, soit évacué par le canal de sortie 13 du dispositif 1 pour mélanger comme décrit ci-dessous.

Selon un premier mode opérationnel, le mélange est destiné à être stocké dans l'un des premier et second réservoirs 3, 2, pour être utilisé ultérieurement. Dans ce cas, après l'étape 75, on réalise l'étape 78 au cours de laquelle on désolidarise le premier ensemble jetable 4 du second ensemble réutilisable 5 et on retire les premier et/ou second réservoirs 3, 2 des embouts pourvus respectivement des premier et second orifices 6, 7 de sorte à pouvoir conserver le second réservoir 2 contenant le mélange. Les premier et second réservoirs 3, 2 peuvent par ailleurs être prélevés sans désolidariser le premier ensemble jetable 4 du second ensemble réutilisable 5.

Selon un second mode opérationnel, pour évacuer le mélange, l'unité centrale de commande et de contrôle 19 commande à l'étape 76, la rotation de la portion mobile 11 du sélecteur fluidique 9, de sorte à mettre en coïncidence le canal 31 de la portion mobile 11 avec le canal de sortie 13. Le mélange peut alors être évacué du dispositif 1 pour mélanger, et administré au patient selon le protocole. Le mélange peut également être transféré vers tout contenant adapté à son administration ultérieure et/ou à son stockage.

Dans le cas d'une injection à un patient, directement avec le dispositif 1 pour mélanger, le dispositif 1 pour mélanger est placé, à l'étape 77, par l'utilisateur, par exemple le patient lui-même, à l'emplacement sur le patient où le mélange doit être injecté. L'unité centrale de commande et de contrôle 19, en accord avec les informations reçues par les capteurs 19D et de la base de données interne 19C ou externe, commande le déplacement de l'aiguille 14 dans la direction axiale A pour percer la peau du patient jusqu'à la profondeur désirée. La pompe 15 est alors actionnée par l'unité centrale de commande et de contrôle 19 et le mélange est injecté. En fin d'injection, l'unité de commande et de contrôle 19, toujours en accord avec les informations des capteurs 19D et des bases de données, arrête la pompe 15 et commande le retrait de l'aiguille 14.

Le mélange et l'injection étant terminée, l'utilisateur peut à l'étape 78 désolidariser les premier et second ensembles jetable 4 et réutilisable 5, le premier ensemble 4 jetable étant alors traité comme un déchet médical et le second ensemble 5 étant prêt pour réaliser un autre mélange de constituants.

Ainsi, avec le dispositif 1 pour mélanger selon l'invention, l'utilisateur peut facilement mélanger deux constituants et brasser le mélange obtenu pour obtenir une solution homogène sans utiliser d'autres composants que le dispositif 1 pour mélanger lui-même. La manipulation du dispositif 1 pour mélanger selon l'invention est réduite à trois étapes principales qui consistent pour l'utilisateur à premièrement, monter les parties jetables (premier ensemble jetable 4 et premier et second réservoirs 3, 2) sur la partie réutilisable (second ensemble réutilisable 5) ; deuxièmement, actionner l'unité centrale de commande et de contrôle 19 pour déclencher le mélange/brassage entièrement automatisé ; troisièmement, administrer le mélange ou le transférer ou le stocker. Cela limite considérablement le temps passé et les risques d'erreur, mais aussi le risque de blessures, ou de contamination au cas ou la substance active est dangereuse pour l'opérateur, tel que par exemple avec les substances cytotoxiques. De plus, grâce à l'unité centrale de commande et de contrôle 19 qui gère l'actionnement de la pompe 15, le dosage des constituants est très précis et on réalise un bon brassage du mélange.

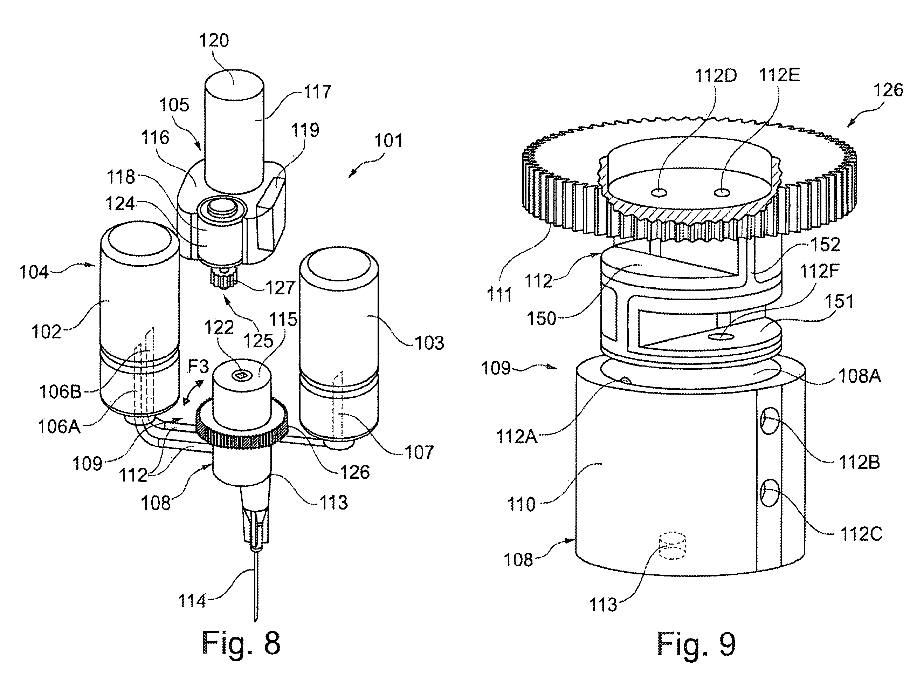

En se référant maintenant aux figures 8 et 9, on va décrire un second mode de réalisation du dispositif pour mélanger selon l'invention. Sur la figure 8, on a ainsi représenté un dispositif 101 pour mélanger selon l'invention, et les éléments communs aux dispositifs 1 et 101 pour mélanger qui remplissent les mêmes fonctions sont affectés du même chiffre augmenté de 100. Par souci de clarté et de concision, nous décrirons ci-dessous essentiellement les éléments du dispositif 101 pour mélanger qui diffèrent du dispositif 1 pour mélanger précédemment décrit.

Comme visible sur la figure 8, le dispositif 101 pour mélanger comprend un socle 108 sur lequel sont montés un second réservoir 102 connecté à un couple de deux embouts pourvus de seconds orifices 106A, 106B de manière étanche, un premier réservoir 103 connecté à un autre embout pourvu d'un premier orifice 107 de manière étanche, ainsi qu'un canal de sortie 113 pour évacuer le mélange par exemple par une aiguille 114 d'injection. Le socle 108 reçoit également un sélecteur fluidique 109 rotatif qui sera décrit plus loin en référence à la figure 9.

Comme on peut aussi le voir sur la figure 8, le dispositif 101 pour mélanger comprend aussi une pompe 115 motorisée montée sur le socle 108, ici de type mono-directionnelle à deux ports (non montrés), adaptée pour faire circuler les constituants dans le sélecteur fluidique 109 entre les premier et second réservoirs 103, 102 et le canal de sortie 113.
Par ailleurs, le dispositif 101 pour mélanger comprend sur un châssis 116 un premier entraînement motorisé 117 de la pompe 115 et un second entraînement motorisé 118 du sélecteur fluidique 109 qui sont commandés par une unité centrale 119 montée sur le châssis 116.

Dans le dispositif 101 pour mélanger, le premier entraînement motorisé 117 de la pompe 115 comprend un premier moteur 120 et un premier ensemble de transmission 121 relié à la pompe 115 qui se présente ici sous la forme d'un emmanchement carré 122. Le premier ensemble de transmission 121 pourrait aussi se présenter sous une autre forme de type verrou permettant l'entraînement de la pompe 115. Le second entraînement motorisé 118 du sélecteur fluidique 109 comprend ici un second moteur 124 et un second ensemble de transmission 125 comportant un couple d'engrenages 126, 127 s'engrenant l'un dans l'autre, l'engrenage 126 étant fixé sur la portion mobile 111 et l'engrenage 127 étant relié au second entraînement motorisé 118. Ainsi, lorsque l'engrenage 127 est entraîné en rotation par le second entrainement motorisé 118, il entraîne l'engrenage 126 en rotation selon la double-flèche F3 indiquée sur la figure 8, de sorte à mettre en coïncidence des canaux du réseau de canaux 112 du sélecteur fluidique 109, comme cela sera décrit plus loin.

Comme on peut mieux le voir sur la figure 9, le socle 108 constitue une portion fixe 110 du sélecteur fluidique 109 sensiblement différent du sélecteur fluidique 9 du dispositif 1 pour mélanger décrit plus haut. Le socle 108, sensiblement cylindrique, est ici creusé d'un évidement 108A aussi sensiblement cylindrique, relié au canal de sortie 113 et apte à recueillir une portion mobile 111 du sélecteur fluidique 109, également sensiblement cylindrique, et d'un réseau de canaux 112 pouvant être mis en coïncidence de sorte à relier sélectivement les embouts pourvus des seconds orifices 106A, 106B, 107 et le canal de sortie 113.

Plus précisément, trois canaux 112A, 112B, 112C sont formés dans la périphérie de la portion fixe 110 du sélecteur fluidique 109, ces canaux 112A, 112B, 112C étant reliés respectivement d'un côté à l'embout pourvu du premier orifice 107 et aux embouts pourvus des seconds orifices 106A, 106B et de l'autre côté débouchant dans l'évidement 108A. Par ailleurs, deux évidements 150, 151 demi-cylindriques perpendiculaires l'un à l'autre sont formés latéralement dans la portion mobile 111 du sélecteur fluidique 109, chaque évidement 150, 151 étant prolongé par un canal respectif 112D, 112E relié à un port de la pompe 115. Un ensemble de joints 152 est disposé autour des évidements 150, 151 pour une parfaite étanchéité du sélecteur fluidique 109.

En outre, la portion mobile 111 a une tête formant l'engrenage 126 de plus grand diamètre que le socle 108 de sorte à pouvoir être entraînée par l'engrenage 127.

En fonctionnement du sélecteur fluidique 109, la portion mobile 111 est insérée dans la portion fixe 110 et peut occuper trois positions angulaires pour relier sélectivement le canal de sortie 113 ou les seconds et premiers orifices 106A, 106B, 107 à la pompe 115 ou entre eux.

Dans une première position angulaire de la portion mobile 111 dans la portion fixe 110, le canal 112A menant à l'embout pourvu du premier orifice 107 du premier réservoir 103 coïncide avec l'évidement 150 et le canal 112D menant à la pompe 115, et le canal 112C menant à l'embout pourvu du second orifice 106A du second réservoir 102 coïncide avec l'évidement 151 et le canal 112E menant à la pompe 115, de sorte à relier fluidiquement les premier et second réservoirs 103, 102 et transvaser un constituant d'un premier, second réservoir 103, 102 à l'autre. Les deux autres canaux 112B et 113 sont obturés dans cette première position.

Dans une deuxième position angulaire de la portion mobile 111, les deux canaux 112B, 112C menant aux embouts pourvus des seconds orifices 106A, 106B du second réservoir 102 coïncident respectivement avec les évidements 150 et 151, de sorte à relier fluidiquement entre eux ces deux embouts pourvus des seconds orifices 106A, 106B, via les canaux 112D, 112E et la pompe 115, pour brasser le mélange dans le second réservoir 102 par exemple. Les canaux 112A et 113 sont obturés dans cette deuxième position. Pour faciliter la circulation fluidique, les embouts portant les seconds orifices 106A, 106B présentent des longueurs différentes et ou des sections terminales de dimensions intérieures différentes. A cet effet et à titre d'exemple, lorsqu'un liquide de viscosité d'environ 1cPo (centipoise) est utilisé, le second orifice utilisé pour l'aspiration présente de préférence une section terminale avec un diamètre d'environ 0.39 mm et le second orifice utilisé pour le refoulement présente une section terminale de diamètre d'environ 0.24 mm, pour un débit de 0.26ml/s dans un réservoir de 5ml disposé au maximum 8 mm sous la surface du liquide présent, par exemple 2.5 ml. Lors de sa réintroduction dans le second réservoir 102, le liquide est ainsi projeté avec force jusqu'à atteindre la paroi du second réservoir 102, ce qui favorise le lavage du second réservoir 102 ainsi que le brassage.

Enfin, dans une troisième position angulaire de la portion mobile 111, le canal 112B menant à l'embout pourvu du second orifice 106B du second réservoir 102 coïncide avec l'évidement 150 et le canal 112D menant à la pompe 115, et le canal 112E de la pompe 115 coïncide avec le canal de sortie 113, de sorte à relier fluidiquement le second réservoir 102 à la sortie et donc évacuer le mélange. Les autres canaux 112A et 112C sont obturés dans cette troisième position.

Le dispositif 101 pour mélanger selon l'invention est de préférence composé de deux ensembles 104, 105 s'emboîtant l'un dans l'autre, le premier ensemble jetable 104 étant conçu pour être stérilisé et à usage unique et le second ensemble réutilisable 105 étant apte à être réutilisé. Ainsi, le premier ensemble jetable 104 comprend les parties fluidiques du dispositif 101 pour mélanger telles que notamment la pompe 115, le sélecteur fluidique 109 et le socle 108, et le second ensemble réutilisable 105 comprend le châssis 116 avec les entraînements motorisés 117, 118 et l'unité centrale de commande et de contrôle 119.

On décrira maintenant en référence à la figure 7 l'utilisation du dispositif 101 pour mélanger selon l'invention pour procéder au mélange d'un constituant de poudre solide ou de lyophilisé contenu dans le second réservoir 102 avec un solvant contenu dans le premier réservoir 103, étant entendu que seules les variations par rapport au dispositif 1 pour mélanger seront soulignées.

Après avoir introduit les premier et second réservoirs 3, 2 sur les embouts pourvus des seconds et premier orifices 106A, 106B, 107 (étape 70), l'utilisateur actionne à l'étape 71 l'unité centrale de commande et de contrôle 119 pour déclencher les étapes 72 à 76 du mélange et du brassage.

A l'étape 72, l'unité centrale commande la rotation de la portion mobile 111 du sélecteur fluidique 109 pour atteindre la première position angulaire décrite ci-dessus. Lorsque, à l'étape 73 d'aspiration, l'unité centrale de commande et de contrôle 119 actionne l'entrainement 117 de la pompe 115, le solvant contenu dans le premier réservoir 103 est aspiré par la pompe 115 et dirigé dans le second réservoir 102.

Puis, à l'étape 74, l'unité centrale de commande et de contrôle 119 commande de nouveau la rotation de la portion mobile 111 du sélecteur fluidique 109, de sorte à atteindre la deuxième position angulaire décrite ci-dessus. Ainsi, à l'étape 75 de brassage, la pompe 115 fait circuler le mélange de poudre solide en suspension ou du constituant lyophilisé réhydraté avec le solvant entre les embouts pourvus des seconds orifices 106A, 106B du second réservoir 102 pour brasser le mélange.

Enfin, selon un premier mode opérationnel, lorsque le mélange est homogène, selon le protocole, l'unité centrale de commande et de contrôle 119 peut commander à l'étape 76 la rotation de la portion mobile 111 du sélecteur fluidique 109, de sorte à atteindre la troisième position angulaire décrite ci-dessus, en vue de l'évacuation du mélange.

Selon un second mode opérationnel, on peut également conserver le mélange obtenu dans le second réservoir 102.

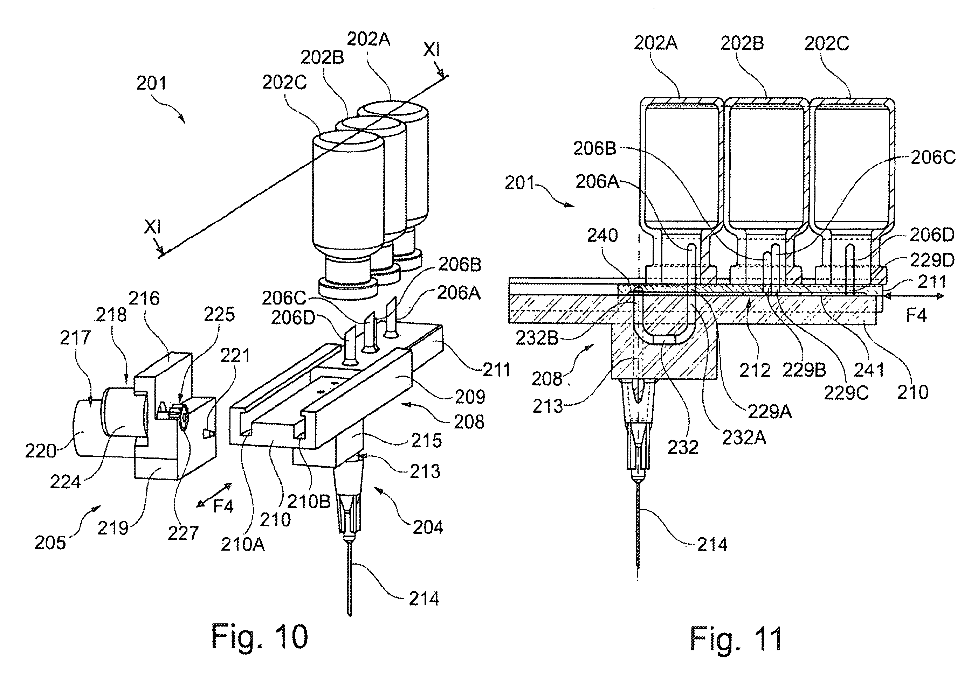

En se référant maintenant aux figures 10 à 13, on va décrire un troisième mode de réalisation du dispositif pour mélanger selon l'invention. Sur la figure 10, on a représenté un dispositif 201 pour mélanger, et les éléments communs aux dispositifs 1 et 201 pour mélanger qui remplissent les mêmes fonctions sont affectés du même chiffre augmenté de 200. Ainsi, comme précédemment, on décrira ci-dessous principalement les éléments du dispositif 201 pour mélanger qui diffèrent du dispositif 1 pour mélanger décrit plus haut.

Le dispositif 201 pour mélanger est conçu pour mélanger et brasser des constituants dans des premier, deuxième et troisième réservoirs 202A, 202B, 202C médicaux standards, ici au nombre de trois.

Comme on peut le voir sur la figure 10, le dispositif 201 pour mélanger comprend un socle 208 portant un coulisseau mobile 211 coulissant selon une direction F4 indiquée sur la figure 10 dans une glissière fixe 210, qui forment ensemble un sélecteur fluidique 209 linéaire à un réseau de canaux 212.

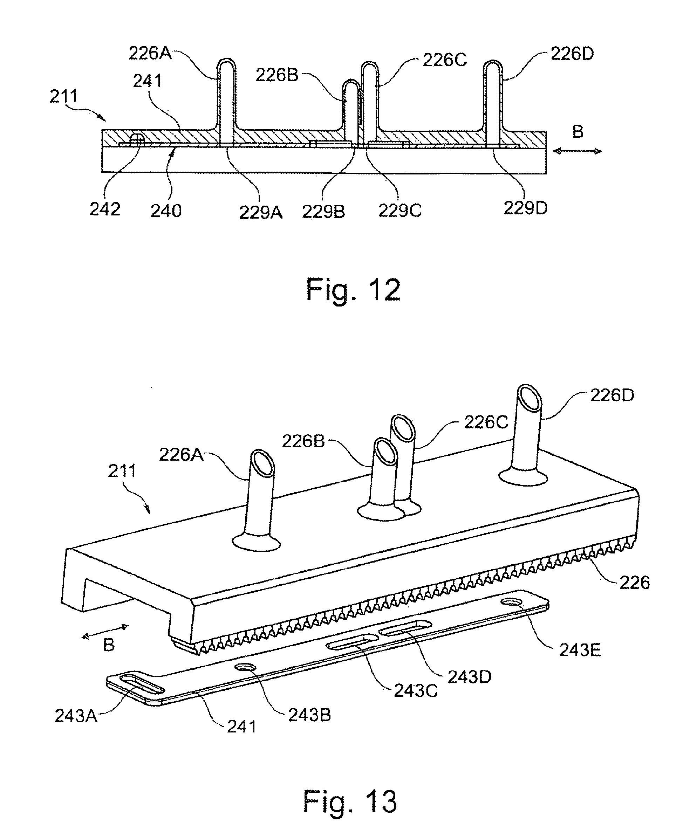

Plus précisément, le coulisseau 211 a un profil en U inversé qui coulisse dans deux rainures 210A, 210B de la glissière 210 en forme de E couché. Le coulisseau 211 est ici percé sur la base du U de quatre orifices 229A, 229B, 229C, 229D traversants alignés selon la direction F4, mieux visibles sur la figure 12, qui sont prolongés respectivement par quatre embouts, pourvus des premier, seconds et troisième orifices 206A, 206B, 206C, 206D, et aptes à recevoir les premier, deuxième et troisième réservoirs 202A, 202B, 202C de manière étanche comme cela sera décrit plus bas. Comme représenté sur la figure 11, les embouts pourvus des seconds orifices 206B, 206C pour le second réservoir 202B présentent avantageusement des différences de longueur et/ou de dimensions de section terminales intérieures (par exemple de diamètres intérieurs) pour améliorer la circulation fluidique dans le second réservoir 202B.

La glissière 210 est percée d'un canal de sortie 213 pour évacuer le mélange, ici par une aiguille 214 d'injection mobile par rapport au socle 208. Comme on le verra plus particulièrement en référence à la figure 11, les embouts pourvus des premier, seconds, troisième orifices 206A, 206B, 206C, 206D et le canal de sortie 213 peuvent être reliés sélectivement par le réseau de canaux 212 du sélecteur fluidique 209.

De manière similaire aux dispositifs 1 et 101 pour mélanger précédemment décrits, le dispositif 201 pour mélanger comprend, montée sur le socle 208, une pompe 215 motorisée, ici de type bi-directionnelle à deux ports (non visibles), adaptée pour faire circuler les constituants dans le réseau de canaux 212, entre les embouts pourvus des premier, seconds, troisième orifices 206A, 206B, 206C, 206D et le canal de sortie 213.

Comme on peut le voir sur la figure 10, le dispositif 201 pour mélanger comprend sur un châssis 216 un premier entraînement motorisé 217 de la pompe 215 et un second entraînement motorisé 218 du sélecteur fluidique 209 qui sont commandés par une unité centrale de commande et de contrôle 219 montée sur le châssis 216.

Plus particulièrement, le premier entraînement motorisé 217 de la pompe 215 comprend un premier moteur 220 et un premier ensemble de transmission 221 relié à la pompe 215, qui se présente ici sous la forme d'un emmanchement carré. Le second entraînement motorisé 218 du sélecteur fluidique 209 comprend un second moteur 224 et un second ensemble de transmission 225 relié au sélecteur fluidique 209, qui se présente ici sous la forme d'un engrenage 227 relié au moteur 224 et qui coopère avec une crémaillère 226 (visible sur la figure 13) formée sur le coulisseau 211 du sélecteur fluidique 209. Ainsi, quand l'entrainement motorisé 218 entraîne en rotation l'engrenage 227, le coulisseau 211 coulisse selon la double-flèche F4 pour mettre en coïncidence les différents canaux du réseau de canaux 212 sélectivement, comme cela sera décrit plus loin.
On comprendra que le dispositif201 selon l'invention est formé de deux ensembles distincts s'emboîtant l'un dans l'autre et détachables, un premier ensemble jetable 204 comprenant les parties du dispositif 201 pour mélanger qui sont en contact avec les constituants à mélanger, à savoir notamment le socle 208, le sélecteur fluidique 209, et la pompe 215, et un second ensemble réutilisable 205 comprenant notamment les entrainements motorisés 217, 218 respectifs de la pompe 215 et du sélecteur fluidique 209.

Sur la figure 11, le dispositif 201 pour mélanger est représenté en coupe pour faire apparaître le réseau de canaux 212 du dispositif 201 pour mélanger. En particulier, un canal 232, en forme de U, relié à la pompe215 est formé dans la glissière 210, ce canal 232 débouchant en deux orifices 232A, 232B faisant face au coulisseau 211.

On a représenté sur la figure 12 le coulisseau 211 en coupe comportant un évidement 240 central sur une partie de la longueur du coulisseau 211 qui entoure les orifices 229A à 229D et un cinquième orifice borgne 242 oblong aligné avec les orifices 229A à 229D selon la direction F4 (visible sur la figure 11). Dans l'évidement 240, on a représenté une pièce intercalaire 241 de forme complémentaire à l'évidement 240 destinée à être interposée entre le coulisseau 211 et la glissière 210 lorsque le coulisseau 211 est monté dans la glissière 210 et servant de guide fluidique et de joint.

Sur la figure 13, on voit mieux la pièce intercalaire 241 qui est percée de cinq orifices 243A, 243B, 243C, 243D, 243E alignés et coïncidant respectivement avec le trou borgne 242 et les orifices 229A à 229D lorsque la pièce intercalaire 241 est insérée dans l'évidement 240, trois orifices 243A, 243C, 243D étant oblongs et deux orifices 243A, 243D sensiblement circulaires. On voit aussi que la pièce intercalaire 240 forme dans une direction sensiblement perpendiculaire à la direction F4 un retour dans lequel l'orifice 243A oblong s'étend (l'orifice borgne 242 s'étendant dans cette même direction), les deux autres orifices oblongs 243C, 243D s'étendant dans la direction F4.

On comprendra que le dispositif 201 pour mélanger est équipé au niveau des différents canaux du réseau de canaux 212 d'éléments d'étanchéité adaptés non représentés.

Ainsi, lorsque le coulisseau 211 avec la pièce intercalaire 241 est en position dans la glissière 210, les orifices 232A, 232B du canal 232 venant de la pompe 215 peuvent être mis en communication avec deux orifices 242, 229A à 229D adjacents du coulisseau 211 de sorte à relier sélectivement la pompe 215 à l'un ou à deux des premier, deuxième et troisième réservoirs 202A, 202B, 202C et/ou au canal de sortie 213. En particulier, on peut ici définir quatre positions latérales différentes du coulisseau 211 dans la glissière 210.

Dans une position initiale latérale du coulisseau 211 dans la glissière 210 illustrée sur la figure 11, le canal 232 met en communication l'embout pourvu du premier orifice 206A du premier réservoir 202A avec l'orifice borgne 242 du coulisseau 211 qui est relié au canal de sortie 213, de sorte à relier fluidiquement le premier réservoir 202A à la sortie et donc évacuer son contenu. Les autres embouts pourvus des seconds et troisième orifices 206B, 206C, 206D des deux autres deuxième, troisième réservoirs 202B, 202C sont obturés dans cette première position.

Dans une première position latérale du coulisseau 211, le canal 232 met en communication l'embout pourvu du premier orifice 206A du premier réservoir 202A et l'embout pourvu du second orifice 206B du deuxième réservoir 202B, de sorte à relier fluidiquement ces deux premier, deuxième réservoirs 202A, 202B entre eux pour transvaser un constituant d'un premier, second réservoir 202A, 202B à l'autre. Le canal de sortie 213 et les embouts pourvus des second et troisième orifices 206C, 206D sont obturés dans cette deuxième position.

Dans une deuxième position latérale du coulisseau 211, le canal 232 met en communication les deux embouts pourvus des seconds orifices 206B, 206C du deuxième réservoir 202B, pour brasser le mélange dans le deuxième réservoir 202B. Le canal de sortie 213 et les embouts pourvus des seconds orifices 206A, 206D sont obturés dans cette troisième position.

Enfin, dans une troisième position latérale du coulisseau 211, le canal 232 met en communication l'embout 206C du deuxième réservoir 202B et l'embout pourvu du troisième orifice 206D du troisième réservoir 202C, de sorte à relier fluidiquement ces deuxième, troisième réservoirs 202B, 202C entre eux pour transvaser un constituant ou le mélange d'un deuxième, troisième réservoir 202B, 202C à l'autre. Le canal de sortie 213 et les embouts pourvus des premier et second orifices 206A, 206B sont obturés dans cette quatrième position.

L'utilisation du dispositif 201 pour mélanger selon l'invention sera maintenant décrite en référence à la figure 7, seules les variations par rapport au dispositif 1 pour mélanger étant décrites, les étapes principales étant les mêmes que celles décrites plus haut pour le dispositif 1 pour mélanger. Le dispositif 201 pour mélanger peut être utilisé pour procéder au mélange et au brassage d'un constituant sous forme de poudre solide ou de lyophilisé contenu dans le deuxième réservoir 202B avec un solvant contenu dans le premier réservoir 202A, puis évacuer le mélange soit dans un troisième réservoir 202C, soit vers le canal de sortie 213 et l'aiguille 214.

Après avoir introduit les premier, deuxième et troisième réservoirs 202A, 202B, 202C sur les embouts pourvus des premier, seconds et troisième orifices 206A, 206B, 206B, 206D (étape 70), l'utilisateur actionne à l'étape 71 l'unité centrale 219 pour déclencher les étapes 72 à 76 du mélange.

A l'étape 72, l'unité centrale de commande et de contrôle 219 commande le déplacement latéral du coulisseau 211 du sélecteur fluidique 209 pour atteindre la deuxième position latérale décrite ci-dessus. Lorsque, à l'étape 73 d'aspiration, l'unité centrale de commande et de contrôle 219 actionne l'entrainement 217 de la pompe 215, le solvant contenu dans le premier réservoir 202A est aspiré par la pompe 215 et dirigé dans le deuxième réservoir 202B.

Puis, à l'étape 74, l'unité centrale de commande et de contrôle 219 commande de nouveau le déplacement latéral du coulisseau 211, de sorte à atteindre la troisième position latérale décrite ci-dessus. Ainsi, à l'étape 75 de brassage, la pompe 215 fait circuler le mélange entre les deux embouts pourvus des seconds orifices 206B, 206C du deuxième réservoir 202B pour brasser le mélange dans ce deuxième réservoir 202B.

Lorsque le mélange est homogène, l'unité centrale de commande et de contrôle 219 commande à l'étape 76 le déplacement latéral du coulisseau 211, de sorte à atteindre la troisième position latérale décrite ci-dessus, en vue de l'administration du mélange soit directement par le canal de sortie 213, soit indirectement par le troisième réservoir 202C.

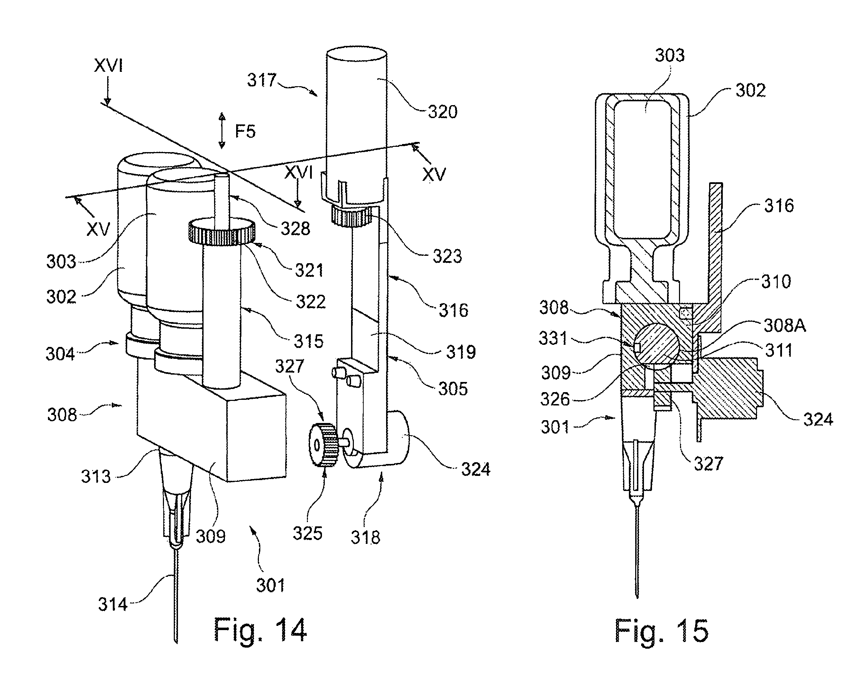

On va maintenant décrire un quatrième mode de réalisation du dispositif pour mélanger selon l'invention en se référant maintenant aux figures 14 à 16. Sur la figure 14, on a représenté un dispositif 301 pour mélanger, et les éléments communs aux dispositifs 1 et 301 pour mélanger qui remplissent les mêmes fonctions sont affectés du même chiffre augmenté de 300. Ainsi, comme précédemment, on décrira ci-dessous principalement les éléments du dispositif 301 pour mélanger qui diffèrent du dispositif 1 pour mélanger décrit plus haut.

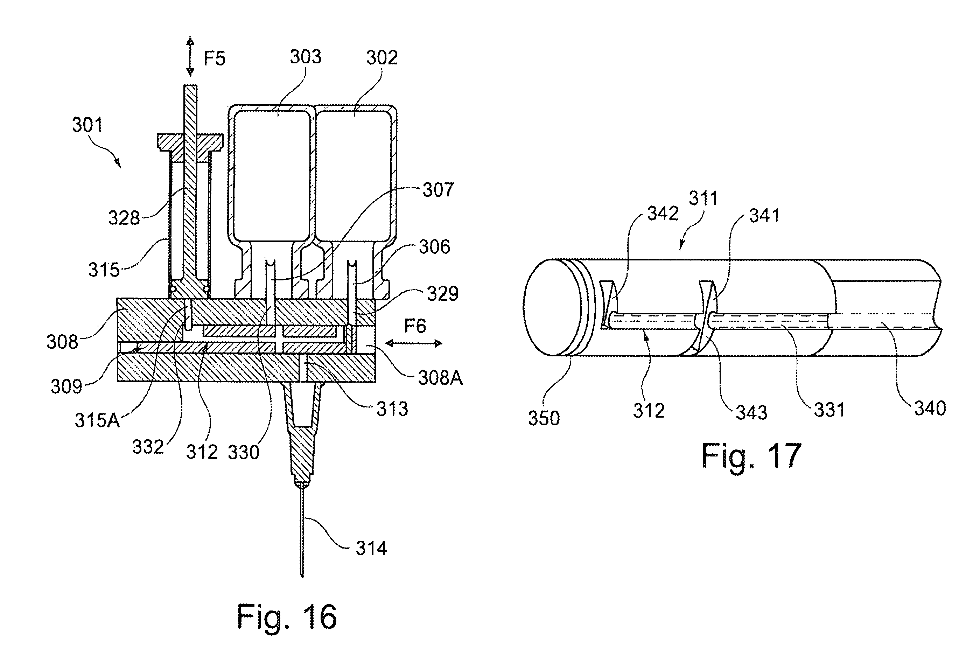

Sur la figure 14, on a représenté un dispositif 301 pour mélanger comprenant un socle 308 sur lequel sont montés un premier et un second réservoirs 303, 302 connectés de manière étanche chacun à un embout pourvu d'un premier, second orifices 307, 306 respectif (visibles sur la figure 16) et comportant un canal de sortie 313 pour évacuer le mélange par une aiguille 314 d'injection. Le socle 108 comprend également un sélecteur fluidique 309 linéaire comprenant un réseau de canaux 312 pouvant être mis en coïncidence de sorte à relier sélectivement les premier et second réservoirs 303, 302 et le canal de sortie 313, comme cela sera décrit plus loin en référence aux figures 15 et 16.

Le dispositif 301 pour mélanger comprend aussi, montée sur le socle 308, une pompe 315 motorisée, ici de type pompe bi-directionnelle à piston 328 avec un port unique 315A visible sur la figure 16, adaptée pour faire circuler les constituants dans le sélecteur fluidique 309, entre les embouts pourvus d'un premier, second orifices 306, 307 et le canal de sortie 313.

Comme on peut aussi le voir sur la figure 14, le dispositif 301 pour mélanger comprend un châssis 316 sur lequel sont montés un premier entraînement motorisé 317 de la pompe 315 et un second entraînement motorisé 318 du sélecteur fluidique 309, commandés par une unité centrale de commande et de contrôle 319 montée aussi sur le châssis 316.

Plus particulièrement, le premier entraînement motorisé 317 de la pompe 315 comprend un premier moteur 320 et un premier ensemble de transmission 321 relié à la pompe 315, qui se présente ici sous la forme d'un couple d'engrenages 322, 323 qui s'engrènent l'un dans l'autre, l'un 322 relié à la pompe 315 et l'autre 323 relié au premier entraînement motorisé 317.

De manière similaire, le second entraînement motorisé 318 du sélecteur fluidique 309 comprend un second moteur 324 et un second ensemble de transmission 325 relié au sélecteur fluidique 309, qui se présente ici sous la forme d'un engrenage 327 relié au moteur 324 et coopérant avec une crémaillère 326 (visible sur la figure 13) formée sur le sélecteur fluidique 209.

Ainsi, lorsque l'entrainement motorisé 317 entraîne en rotation les engrenages 322, 323, le piston 328 se déplace longitudinalement dans la pompe 315 dans un sens ou l'autre selon la double-flèche F5 indiquée sur la figure 14 pour faire circuler les constituants dans le dispositif 301 pour mélanger.

Comme on peut le voir sur la figure 15, le socle 308 forme une portion fixe 310 d'un sélecteur fluidique 309 linéaire et est creusé d'un évidement 308A sensiblement cylindrique de sorte à recevoir une portion mobile 311 sensiblement cylindrique du sélecteur fluidique 309. Ainsi, quand l'entrainement motorisé 318 entraîne en rotation l'engrenage 327, la crémaillère 326 formée sur la portion mobile 311 est entraînée selon la double-flèche F6 indiquée sur la figure 16 pour mettre en coïncidence les différents canaux du réseau de canaux 312 du sélecteur fluidique 309, comme cela sera décrit plus loin.

Avantageusement, le dispositif 301 pour mélanger peut aussi être conformé en deux ensembles 304, 305 distincts s'emboîtant l'un dans l'autre, comme cela apparaît clairement sur la figure 14, le premier ensemble jetable 304 comprenant toutes les parties du dispositif 1 pour mélanger qui sont en contact avec les constituants à mélanger (notamment pompe 315, sélecteur fluidique 309, socle 308) et le second ensemble réutilisable 305 comprenant le reste du dispositif 301 pour mélanger (notamment châssis 316, entraînements motorisés 317, 318).

Comme on peut le voir sur la figure 16, le socle 308 est creusé de deux premiers canaux 329, 330 reliés respectivement directement aux embouts pourvus des second et premier orifices 306, 307 et débouchant dans l'évidement 308A du socle 308. Par ailleurs, le socle 308 est creusé d'un troisième canal 332 mettant en communication le port 315A de la pompe 315 et l'évidement 308A.

On a représenté sur la figure 17 la portion mobile 311 qui est creusée longitudinalement sur sa périphérie d'un canal 331 qui débouche latéralement sur quatre portions de canaux 340, 341, 342, 343 sensiblement perpendiculaires au canal 331, une première portion de canal 340 très large étant destinée à communiquer avec la pompe 315 et les autres portions de canaux 341, 342, 323 étant destinés à relier chacune un des canaux 329, 330 conduisant aux deuxième, troisième réservoirs 302, 303 ou du canal de sortie 313.

En outre, la portion mobile 311 est équipée à une extrémité d'un joint 350 pour fermer de manière étanche l'évidement 308A du sélecteur fluidique 309. On comprendra que le dispositif 301 pour mélanger est équipé au niveau des différents canaux du réseau de canaux 312 d'éléments d'étanchéité adaptés non représentés.

Lorsque la portion mobile 311 est en position dans l'évidement 308A comme représenté sur la figure 16, elle peut occuper trois positions latérales différentes selon la direction indiquée par la flèche F6 sur la figure 16. Dans ces trois positions latérales, la portion de canal 340 reste en communication avec le canal 332 reliant la pompe 315, de sorte que la pompe 315 communique alternativement avec un des premier, second réservoirs 303, 302 ou le canal de sortie 313.

Dans une première position latérale de la portion mobile 311 dans la portion fixe 310, la portion de canal 342 de la portion mobile 311 coïncide avec le canal 329 menant à l'embout pourvu du premier orifice 307 du premier réservoir 303, de sorte que la pompe 315 est reliée fluidiquement au premier réservoir 303. On comprendra que les deux autres canaux 341, 343 sont obturés dans cette première position.

Dans une deuxième position latérale de la portion mobile 311, la portion de canal 341 de la portion mobile 311 coïncide avec le canal 330 menant à l'embout pourvu du premier orifice 307 du second réservoir 302, de sorte que la pompe 315 est reliée fluidiquement au second réservoir 302. On comprendra que dans cette deuxième position les deux autres canaux 342, 343 sont obturés.

Enfin, dans une troisième position latérale de la portion mobile 311, la portion de canal 343 de la portion mobile 311 coïncide avec le canal de sortie 313, de sorte que la pompe 315 est reliée fluidiquement à la sortie pour évacuer le mélange. On comprendra que dans cette troisième position, les deux autres canaux 341, 342 sont obturés.

L'utilisation du dispositif 301 pour mélanger selon l'invention sera maintenant décrite en référence à la figure 7, étant entendu que seules les variations par rapport au dispositif 1 pour mélanger seront décrites, les étapes principales étant les mêmes que celles décrites plus haut pour le dispositif 1 pour mélanger. Le dispositif 301 pour mélanger peut être utilisé pour procéder au mélange et au brassage d'un constituant de poudre solide ou de lyophilisé contenu dans le second réservoir 302 avec un solvant contenu dans le premier réservoir 303, puis évacuer le mélange vers le canal de sortie 313 et l'aiguille 314.

Comme décrit précédemment en relation avec le dispositif 1 pour mélanger, l'utilisateur introduit les premier, second réservoirs 303, 302 sur les embouts pourvus des premier et second orifices 307, 306 respectifs à l'étape 70, puis actionne à l'étape 71 l'unité centrale 319 pour déclencher les étapes 72 à 76 du mélange.

A l'étape 72, l'unité centrale de commande et de contrôle 319 commande le déplacement latéral de la portion mobile 311 du sélecteur fluidique 309 pour atteindre la première position latérale décrite ci-dessus. Puis, à l'étape 73 d'aspiration, l'unité centrale de commande et de contrôle 319 actionne la pompe 315 pour aspirer le solvant contenu dans le premier réservoir 303 dans la pompe 315.
Puis, à l'étape 74, l'unité centrale de commande et de contrôle 319 commande le déplacement latéral de la portion mobile 311 pour atteindre la deuxième position latérale décrite ci-dessus. Ainsi, à l'étape 75 de brassage, la pompe 315 refoule le solvant dans le second réservoir 302, puis on procède au brassage du mélange jusqu'à homogénéisation complète en actionnant la pompe 315.

Lorsque le mélange est homogène, selon un premier mode opérationnel, l'unité centrale de commande et de contrôle 319 peut commander à l'étape 76 le déplacement latéral de la portion mobile 311 pour atteindre la troisième position latérale décrite ci-dessus, en vue de l'administration du mélange directement par le canal de sortie 213. Selon un second mode opérationnel, on peut également conserver le mélange obtenu dans le second réservoir 302.

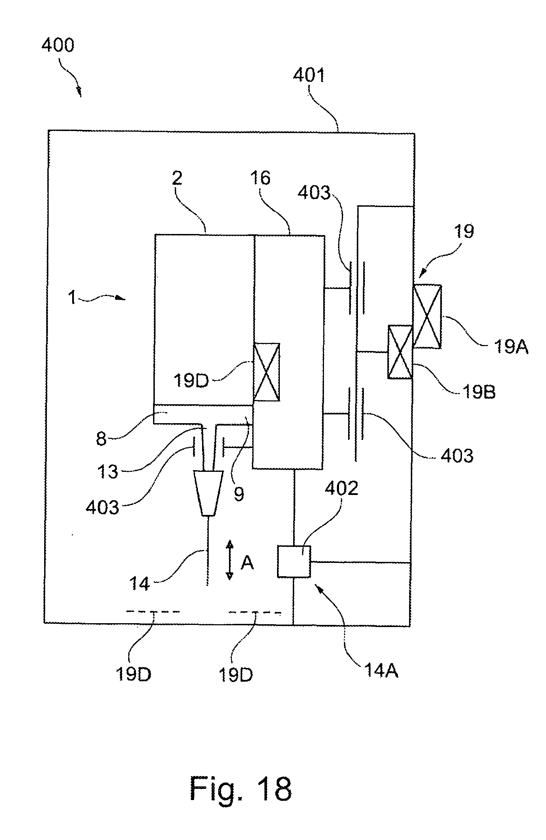

La figure 18 présente de manière schématique un dispositif d'injection 400 miniaturisé à usage médical comprenant dans une enceinte 401 un dispositif 1 pour mélanger selon l'invention pour mélanger de manière homogène des constituants d'un mélange et permettre d'injecter ce mélange à un patient via l'aiguille 14 d'injection.

Sur la figure 18, on a représenté le second réservoir 2 du dispositif 1 pour mélanger monté sur le socle 8 et relié via le sélecteur fluidique 9 au canal de sortie 13 menant à l'aiguille 14 d'injection. Le socle 8 est relié au châssis 16 comme cela est décrit plus haut en relation avec les figures 1 à 3.

Comme visible sur la figure 18, les moyens de déplacement 14A de l'aiguille 14 comprennent un actuateur 402, par exemple sous la forme d'un moteur et d'un ensemble tige-écrou ou d'une crémaillère ou sous la forme d'un ensemble de ressorts, pour déplacer l'aiguille 14 par rapport à l'enceinte 401 grâce à un ou plusieurs moyens de guidage 403, par exemple des glissières, des moyens de déplacement 14A. Plus précisément, l'ensemble composé de l'aiguille 14, du socle 8 et du châssis 16 avec tous les éléments montés sur ceux-ci est monté mobile par rapport à l'enceinte 401 selon la direction axiale A.

On comprendra que pour une meilleure ergonomie du dispositif d'injection 400 une partie de l'unité centrale de commande et de contrôle 19 pourra être montée directement sur l'enceinte 401, comme par exemple l'interface utilisateur 19A et/ou l'alimentation 19B, comme cela est représenté sur la figure 18.

Par ailleurs, on peut prévoir dans l'enceinte 401 un support (non représenté) des second et premier réservoirs 2, 3 de sorte que les moyens de déplacement 14A peuvent aussi permettre une perforation automatique des second et premier réservoirs 2, 3, par exemple par les embouts pourvus des second et premier orifices 6, 7, grâce à un déplacement du socle 8 par rapport à l'enceinte 401 selon la direction axiale A.

Ainsi, en vue d'une injection d'un mélange de constituant à l'aide d'un dispositif d'injection 400 selon l'invention, l'utilisateur procède d'abord à un mélange des constituants en réalisant les étapes 70 à 76 décrites plus haut en relation avec la figure 7, puis il actionne à l'étape 77 les moyens de déplacement 14A pour réaliser l'injection du mélange obtenu.

Ainsi, grâce au dispositif d'injection 400 selon l'invention, on dispose d'un seul dispositif permettant à la fois de mélanger des constituants d'un mélange de manière simple, précise et automatique, et d'administrer facilement le mélange obtenu à un patient, sans que ces opérations ne nécessitent une formation particulière de l'utilisateur.

Bien entendu, le dispositif d'injection 400 peut tout à fait être adapté pour recevoir un dispositif 101, resp. 201, resp. 301 pour mélanger selon l'invention tel que décrit plus haut en relation avec les figures 8 et 9, resp. 10 à 13, resp. 14 à 17.

Il va de soi que la présente invention ne saurait être limitée à la description qui précède des modes de réalisation décrits ci-dessus, susceptibles de subir quelques modifications sans pour autant sortir du cadre de l'invention.

Ainsi, en variante, au lieu de l'aiguille, le canal de sortie peut être relié à une canule pour goutte à goutte, à un dispositif ambulatoire, à un autre flacon, ou tout autre moyen d'administration du mélange.

Par ailleurs, la pompe pourrait être une pompe à membrane actionnée par un électroaimant, ou pourrait être actionnée par un dispositif piézoélectrique ou tout autre dispositif d'actionnement servant à transformer une énergie quelconque en énergie mécanique.

De plus, on pourrait tout à fait prévoir un sélecteur fluidique manuel réglable simplement par l'utilisateur.

En outre, on pourra facilement adapter les dispositifs 1, 101, 201, 301 pour mélanger selon l'invention en ajoutant un ou plusieurs embouts pourvus d'orifices pour d'autres flacons en vue d'autres applications, et en adaptant en conséquence le sélecteur fluidique. Dans ce cas, on pourra aussi aménager une prise à l'air dans au moins un canal du sélecteur afin de faciliter les opérations de transvasement de volumes de fluide dans des petits réservoirs.

En particulier, l'unité centrale de commande et de contrôle pourra être adaptée pour déclencher des étapes 76 et 77 adaptée à différentes possibilités d'administration du mélange : injection directe, voie orale, stockage temporaire du mélange en vue d'une administration différée, etc. Pour cela, le sélecteur fluidique pourra être adapté pour disposer d'une position de blocage fluidique afin d'éviter toute fuite du mélange.

Par exemple, on pourra stocker le mélange de constituants dans un des réservoirs pour une administration ultérieure au lieu de l'évacuer vers le canal de sortie. On pourra aussi prévoir de remplir à partir du dispositif pour mélanger selon l'invention une série de réservoirs de petite taille avec une partie seulement du mélange de constituants, pour administration à plusieurs patients par exemple. Cela est particulièrement utile en contexte hospitalier.

Par ailleurs, l'unité centrale de commande et de contrôle peut avantageusement être programmable de sorte à réaliser le mélange des constituants à une heure choisie, selon une séquence et des paramètres adaptés aux constituants et aux conditions ambiantes. On pourra alors par exemple prévoir avantageusement une alarme pour indiquer à l'utilisateur que le mélange est prêt.

De plus, la vitesse de la pompe peut avantageusement être réglable pour s'adapter au mélange de constituants variés et notamment éviter la formation de mousse ou d'agglomérats. On peut aussi prévoir un dispositif vibratoire par exemple mécanique ou à ultra-sons, ou un mixeur à turbulence interposé le long d'un des canaux fluidiques, ou encore un filtre monté sur la pompe, pour empêcher la formation d'agglomérats.

Enfin le dispositif pour mélanger peut comprendre plusieurs pompes et/ou plusieurs sélecteurs fluidiques, chaque pompe et chaque sélecteur pouvant être dédiés à un mélange spécifique.

L'invention permet d'atteindre les objectifs précédemment mentionnés tout en apportant des avantages importants.

Tout d'abord, dans le cas d'une administration directe par le dispositif 1, 101, 201, 301 pour mélanger de l'invention, par exemple une injection avec le dispositif d'injection 400 selon l'invention, on utilise très avantageusement une pompe unique pour réaliser à la fois le mélange, le brassage et l'administration d'un produit obtenu par mélange de deux ou plus de constituants.

De plus, au moment de l'administration du mélange, l'ensemble des circuits fluidiques et de la pompe est amorcé avec un mélange homogène permettant un dosage du constituant actif extrêmement précis dans le dispositif selon l'invention, et limitant l'impact négatif du volume mort des dispositifs connus.

## Revendications

1. Dispositif (1 ;101 ; 201 ; 301) pour mélanger au moins deux constituants provenant d'au moins un premier réservoir (3 ; 103 ; 202A ; 303) et un second réservoir (2; 102 ; 202B ; 302) distinct dudit premier réservoir (3 ; 103 ; 202A ;303), ledit dispositif (1 ; 101 ; 201 ; 301) pour mélanger comprenant au moins un premier orifice (7 ; 107 ; 206A ; 307) apte à être raccordé audit premier réservoir (3 ; 103 ; 202A ; 303), et au moins un second orifice (6 ; 106A, 106B ; 206B, 206C ; 306) distinct dudit premier orifice (7 ; 107 ; 206A ; 307) et apte à être raccordé audit second réservoir (2; 102 ; 202B ; 302) et une pompe (15 ; 115 ; 215 ; 315) motorisée, **caractérisé en ce qu'**il comprend un sélecteur fluidique (9 ; 109 ; 209 ; 309) raccordé à ladite pompe (15 ; 115 ; 215 ; 315) et comprenant un réseau de canaux (12 ; 112 ; 212 ; 312), ledit sélecteur fluidique (9 ; 109 ; 209 ; 309) étant pourvu d'au moins :
- une portion fixe (10 ; 110 ; 210 ; 310) comprenant au moins une partie dudit réseau de canaux (12 ; 112 ; 212 ; 312) débouchant au moins par ledit premier orifice (7 ; 107 ; 206A ; 307) et par ledit second orifice (6 ; 106A, 106B ; 206B, 206C ; 306),
- une portion mobile (11, 111 ; 211 ; 311) comprenant au moins une autre partie dudit réseau de canaux (12 ; 112 ; 212 ; 312) et mobile entre plusieurs positions comprenant au moins :
- une première position dans laquelle ledit réseau de canaux (12 ; 112 ; 212 ; 312) met en communication l'un au moins desdits premier orifice (7 ; 107 ; 206A ; 307) et second orifice (6 ; 106A, 106B ; 206B, 206C ; 306) et ladite pompe (15 ; 115 ;215 ; 315) pour permettre, à l'aide de ladite pompe (15 ; 115 ; 215 ; 315), un transfert d'au moins un constituant dudit premier orifice (7 ; 107 ; 206A ; 307) vers ledit second orifice (6 ; 106A, 106B ; 206B, 206C ; 306) par l'intermédiaire de ladite pompe (15 ; 115 ;215 ; 315),
- une seconde position dans laquelle ledit réseau de canaux (12 ; 112 ; 212 ; 312) met en communication ledit second orifice (6 ; 106A, 106B ; 206B, 206C ; 306) et ladite pompe (15 ; 115 ; 215 ;315) pour permettre à l'aide de ladite pompe (15 ; 115 ; 215 ; 315), un brassage par circulation dans ledit réseau de canaux (12 ; 112 ; 212 ; 312) dudit mélange obtenu.

2. Dispositif (1 ; 301) pour mélanger selon la revendication 1, **caractérisé en ce que** ladite pompe (15 ; 315) est au moins apte à être couplée à un réservoir de pompe.

3. Dispositif (101 ; 201) pour mélanger selon l'une des revendications précédente, **caractérisé en ce qu'**il comporte deux seconds orifices (106A, 106B ; 206B, 206C) aptes à être couplés audit second réservoir (102 ; 202B) de sorte que dans ladite seconde position, ledit brassage est obtenu par circulation dudit mélange entre lesdits seconds orifices (106A, 106B ; 206B, 206C) par ladite pompe (115, 215).

4. Dispositif (1 ;101 ;201 ;301) pour mélanger selon au moins l'une des revendications précédentes, **caractérisé en ce que** ledit réseau de canaux (12 ;112 ; 212 ; 312) dudit sélecteur fluidique (9 ;109 ; 209 ; 309) comporte au moins un canal de sortie (13 ; 113, 213 ; 313) pour évacuer ledit mélange dudit dispositif (1 ;101 ; 201 ; 301), **en ce que** ladite portion mobile (11 ; 111 ; 211 ; 311) dudit sélecteur fluidique (9 ; 109 ; 209 ; 309) peut occuper une troisième position dans laquelle ledit réseau de canaux (12 ;112 ; 212 ; 312) met en communication ladite pompe (15 ; 115 ; 215 ; 315) ou ledit second orifice (6 ; 106A, 106B ; 206B, 206C ; 306) audit canal de sortie (13 ; 113, 213 ; 313) pour évacuer ledit mélange dudit dispositif (1 ; 101 ; 201 ; 301) pour mélanger.

5. Dispositif (201) pour mélanger selon l'une des revendications précédentes, **caractérisé en ce qu'**il comporte au moins un troisième orifice (206D) apte à être raccordé à un troisième réservoir (202C) distinct desdits premier et second réservoirs (202B, 202A), **en ce que** ladite portion mobile (211) dudit sélecteur fluidique (209) est apte à être dans une troisième position dans laquelle ledit réseau de canaux (212) met en communication ledit second orifice (206B, 206C) et ledit troisième orifice (206D) pour permettre le transfert dudit mélange dudit second orifice (206B, 206C) vers ledit troisième orifice (206D).

6. Dispositif (1 ;101 ;201 ;301) pour mélanger selon au moins l'une des revendications précédentes, **caractérisé en ce que** l'un au moins desdits premier orifice (7 ; 107 ; 206A ; 307), second orifice (6 ; 106A, 106B ; 206B, 206C ; 306) et troisième orifice (206D) est formé individuellement sur un embout et **en ce que**, lorsque ledit dispositif (1 ; 101 ; 201 ; 301) pour mélanger comporte deux seconds orifices (106A, 106B ; 206B, 206C), les embouts portant ces derniers présentent des longueurs différentes, permettant d'améliorer la circulation fluidique dans ledit second réservoir (2 ; 102 ; 202B ; 302).

7. Dispositif (1 ;101 ;201 ;301) pour mélanger selon au moins l'une des revendications précédentes, **caractérisé en ce que** l'un au moins desdits premier orifice (7 ; 107 ; 206A ; 307), second orifice (6 ; 106A, 106B ; 206B, 206C ; 306) et troisième orifice (206D) est formé individuellement sur un embout et **en ce que**, lorsque ledit dispositif (1 ; 101 ; 201 ; 301) pour mélanger comporte deux seconds orifices (106A, 106B ; 206B, 206C) présentant des sections terminales de dimensions intérieures entre elles, permettant d'améliorer la circulation fluidique dans ledit second réservoir (2 ; 102 ; 202B ; 302).

8. Dispositif (1 ; 101 ; 201 ; 301) pour mélanger selon au moins l'une des revendications précédentes, **caractérisé en ce que** ledit sélecteur fluidique (9 ; 109 ; 209 ; 309) est agencé pour être couplé à un entraînement (18 ; 118 ; 218 ; 318) motorisé apte à le déplacer entre lesdites première, seconde, troisième positions.

9. Dispositif (1 ;101 ;201 ;301) pour mélanger selon au moins la revendication 8, **caractérisé en ce qu'**il comporte un premier ensemble jetable (4 ; 104 ; 204 ; 304) comportant ledit sélecteur fluidique (9 ;109 ; 209 ;309), et un second ensemble réutilisable (5; 105 ; 205 ; 305) comportant ledit entraînement (18 ; 118 ; 218 ; 318) motorisé de ladite pompe (15 ; 115 ; 215 ;315) et dudit sélecteur fluidique (9 ;109 ; 209 ;309), ledit premier ensemble jetable (4 ; 104 ; 204 ; 304) et ledit second ensemble réutilisable (5 ; 105 ; 205 ; 305) étant agencés pour être connectables et détachables respectivement l'un de l'autre.

10. Dispositif (1 ; 101 ; 201 ; 301) pour mélanger selon au moins la revendication 9, **caractérisé en ce que** ledit premier ensemble jetable (4 ; 104; 204 ; 304) comprend au moins ledit premier réservoir (3 ; 103 ; 202A ;303) contenant un solvant et ledit second réservoir (2; 102 ; 202B ; 302) contenant un une poudre solide ou un lyophilisé.

11. Dispositif (1 ;101) pour mélanger selon l'une des revendications 1 à 10, **caractérisé en ce que** ladite portion mobile (11, 111) dudit sélecteur fluidique (9 ; 109) est agencée pour être mobile en rotation par rapport à ladite une portion fixe (10 ; 110).

12. Dispositif (201 ; 301) pour mélanger selon l'une des revendications 1 à 9, **caractérisé en ce que** ladite portion mobile (211 ; 311) sélecteur fluidique (209 ; 309) est agencé pour être mobile en translation par rapport à ladite portion fixe (210 ; 310).

13. Dispositif d'injection (400) miniaturisé à usage médical, **caractérisé en ce qu'**il comprend un dispositif (1 ; 101 ; 201 ; 301) pour mélanger selon l'une des revendications précédentes et une aiguille (14 ; 114 ; 214 ; 314) raccordée audit sélecteur fluidique (9 ; 109 ; 209 ; 309).

14. Dispositif d'injection (400) selon la revendication précédente, **caractérisé en ce que** ladite aiguille (14 ; 114 ; 214 ; 314) est montée mobile dans une enceinte (401) dudit dispositif d'injection (400).

## Patentansprüche

1. Vorrichtung (1; 101; 201; 301) zum Mischen von mindestens zwei Bestandteilen, die aus mindestens einem ersten Behälter (3; 103; 202A; 303) und einem zweiten Behälter (2; 102; 202B; 302), der von dem ersten Behälter (3; 103; 202A; 303) verschieden ist, stammen, wobei die Vorrichtung (1; 101; 201; 301) zum Mischen mindestens eine erste Öffnung (7; 107; 206A; 307), die geeignet ist, mit dem ersten Behälter (3; 103; 202A; 303) verbunden zu werden, und mindestens eine zweite Öffnung (6; 106A, 106B; 206B, 206C; 306), die von der ersten Öffnung (7; 107; 206A; 307) verschieden ist und geeignet ist, mit dem zweiten Behälter (2; 102; 202B; 302) verbunden zu werden, und eine motorbetriebene Pumpe (15; 115; 215; 315) umfasst, **dadurch gekennzeichnet, dass** sie einen Fluidselektor (9; 109; 209; 309) umfasst, der mit der Pumpe (15; 115; 215; 315) verbunden ist und ein Netz von Kanälen (12; 112; 212; 312) umfasst, wobei der Fluidselektor (9; 109; 209; 309) mindestens mit Folgendem versehen ist:
- einem festen Abschnitt (10; 110; 210; 310), der mindestens einen Teil des Netzes von Kanälen (12; 112; 212; 312) umfasst, die zumindest über die erste Öffnung (7; 107; 206A; 307) und über die zweite Öffnung (6; 106A, 106B; 206B, 206C; 306) münden,
- einen beweglichen Abschnitt (11, 111; 211; 311), der mindestens einen anderen Teil des Netzes von Kanälen (12; 112; 212; 312) umfasst und zwischen mehreren Positionen beweglich ist, die mindestens umfassen:
- eine erste Position, in welcher das Netz von Kanälen (12; 112; 212; 312) zumindest eine der ersten Öffnung (7; 107; 206A; 307) und der zweiten Öffnung (6; 106A, 106B; 206B, 206C; 306) und die Pumpe (15; 115; 215; 315) verbindet, um mithilfe der Pumpe (15; 115; 215; 315) eine Überführung wenigstens eines Bestandteils von der ersten Öffnung (7; 107; 206A; 307) zu der zweiten Öffnung (6; 106A, 106B; 206B, 206C; 306) über die Pumpe (15; 115; 215; 315) zu ermöglichen,
- eine zweite Position, in welcher das Netz von Kanälen (12; 112; 212; 312) die zweite Öffnung (6; 106A, 106B; 206B, 206C; 306) und die Pumpe (15; 115; 215; 315) verbindet, um mithilfe der Pumpe (15; 115; 215; 315) ein Mischen der erhaltenen Mischung durch Zirkulation in dem Netz von Kanälen (12; 112; 212; 312) zu ermöglichen.

2. Vorrichtung (1; 301) zum Mischen nach Anspruch 1, **dadurch gekennzeichnet, dass** die Pumpe (15; 315) wenigstens geeignet ist, mit einem Pumpenbehälter gekoppelt zu werden.

3. Vorrichtung (101; 201) zum Mischen nach einem der vorhergehenden Ansprüche, **dadurch gekennzeichnet, dass** sie zwei zweite Öffnungen (106A, 106B; 206B, 206C) aufweist, die geeignet sind, derart mit dem zweiten Behälter (102; 202B) gekoppelt zu werden, dass in der zweiten Position das Mischen durch Zirkulation der Mischung zwischen den zweiten Öffnungen (106A, 106B; 206B, 206C) durch die Pumpe (115, 215) erreicht wird.

4. Vorrichtung (1; 101; 201; 301) zum Mischen nach wenigstens einem der vorhergehenden Ansprüche, **dadurch gekennzeichnet, dass** das Netz von Kanälen (12; 112; 212; 312) des Fluidselektors (9; 109; 209; 309) mindestens einen Ausgangskanal (13; 113, 213; 313) zum Ableiten der Mischung aus der Vorrichtung (1; 101; 201; 301) aufweist, und dadurch, dass der bewegliche Abschnitt (11, 111; 211; 311) des Fluidselektors (9; 109; 209; 309) eine dritte Position einnehmen kann, in welcher das Netz von Kanälen (12; 112; 212; 312) die Pumpe (15; 115; 215; 315) oder die zweite Öffnung (6; 106A, 106B; 206B, 206C; 306) mit dem Ausgangskanal (13; 113, 213; 313) verbindet, um die Mischung aus der Vorrichtung (1; 101; 201; 301) zum Mischen abzuleiten.

5. Vorrichtung (201) zum Mischen nach einem der vorhergehenden Ansprüche, **dadurch gekennzeichnet, dass** sie mindestens eine dritte Öffnung (206D) aufweist, die geeignet ist, mit einem dritten Behälter (202C), der von dem ersten und dem zweiten Behälter (202B, 202A) verschieden ist, verbunden zu werden, und dadurch, dass der bewegliche Abschnitt (211) des Fluidselektors (209) geeignet ist, sich in einer dritten Position zu befinden, in welcher das Netz von Kanälen (212) die zweite Öffnung (206B, 206C) und die dritte Öffnung (206D) verbindet, um die Überführung der Mischung von der zweiten Öffnung (206B, 206C) zu der dritten Öffnung (206D) zu ermöglichen.

6. Vorrichtung (1; 101; 201; 301) zum Mischen nach wenigstens einem der vorhergehenden Ansprüche, **dadurch gekennzeichnet, dass** zumindest eine der ersten Öffnung (7; 107; 206A; 307), der zweiten Öffnung (6; 106A, 106B; 206B, 206C; 306) und der dritten Öffnung (206D) einzeln an einem Anschlussstück ausgebildet ist, und dadurch, dass, wenn die Vorrichtung (1; 101; 201; 301) zum Mischen zwei zweite Öffnungen (106A, 106B; 206B, 206C) aufweist, die Anschlussstücke, welche diese Letzteren tragen, unterschiedliche Längen aufweisen, was es ermöglicht, die Fluidzirkulation in dem zweiten Behälter (2; 102; 202B; 302) zu verbessern.

7. Vorrichtung (1; 101; 201; 301) zum Mischen nach wenigstens einem der vorhergehenden Ansprüche, **dadurch gekennzeichnet, dass** zumindest eine der ersten Öffnung (7; 107; 206A; 307), der zweiten Öffnung (6; 106A, 106B; 206B, 206C; 306) und der dritten Öffnung (206D) einzeln an einem Anschlussstück ausgebildet ist, und dadurch, dass, wenn die Vorrichtung (1; 101; 201; 301) zum Mischen zwei zweite Öffnungen (106A, 106B; 206B, 206C) aufweist, die untereinander Endbereiche mit Innenabmessungen aufweisen, was es ermöglicht, die Fluidzirkulation in dem zweiten Behälter (2; 102; 202B; 302) zu verbessern.

8. Vorrichtung (1; 101; 201; 301) zum Mischen nach wenigstens einem der vorhergehenden Ansprüche, **dadurch gekennzeichnet, dass** der Fluidselektor (9; 109; 209; 309) dazu eingerichtet ist, mit einem motorischen Antrieb (18; 118; 218; 318) gekoppelt zu werden, der geeignet ist, ihn zwischen den ersten, zweiten und dritten Positionen zu bewegen.

9. Vorrichtung (1; 101; 201; 301) zum Mischen wenigstens nach Anspruch 8, **dadurch gekennzeichnet, dass** sie eine erste, wegwerfbare Einheit (4; 104; 204; 304), die den Fluidselektor (9; 109; 209; 309) umfasst, und eine zweite, wiederverwendbare Einheit (5; 105; 205; 305), die den motorischen Antrieb (18; 118; 218; 318) der Pumpe (15; 115; 215; 315) und des Fluidselektors (9; 109; 209; 309) umfasst, aufweist, wobei die erste, wegwerfbare Einheit (4; 104; 204; 304) und die zweite, wiederverwendbare Einheit (5; 105; 205; 305) dazu eingerichtet sind, miteinander verbindbar und voneinander lösbar zu sein.

10. Vorrichtung (1; 101; 201; 301) zum Mischen wenigstens nach Anspruch 9, **dadurch gekennzeichnet, dass** die erste, wegwerfbare Einheit (4; 104; 204; 304) mindestens den ersten Behälter (3; 103; 202A; 303), der ein Lösungsmittel enthält, und den zweiten Behälter (2; 102; 202B; 302), der ein festes Pulver oder eine gefriergetrocknete Substanz enthält, umfasst.

11. Vorrichtung (1; 101) zum Mischen nach einem der Ansprüche 1 bis 10, **dadurch gekennzeichnet, dass** der bewegliche Abschnitt (11, 111) des Fluidselektors (9; 109) dazu eingerichtet ist, bezüglich des einen festen Abschnitts (10; 110) drehbeweglich zu sein.

12. Vorrichtung (201; 301) zum Mischen nach einem der Ansprüche 1 bis 9, **dadurch gekennzeichnet, dass** der bewegliche Abschnitt (211; 311) des Fluidselektors (209; 309) dazu eingerichtet ist, bezüglich des festen Abschnitts (210; 310) translatorisch beweglich zu sein.

13. Miniaturisierte Einspritzvorrichtung (400) zur medizinischen Verwendung, **dadurch gekennzeichnet, dass** sie eine Vorrichtung (1; 101; 201; 301) zum Mischen nach einem der vorhergehenden Ansprüche und eine Nadel (14; 114; 214; 314), die an den Fluidselektor (9; 109; 209; 309) angeschlossen ist, umfasst.

14. Einspritzvorrichtung (400) nach dem vorhergehenden Anspruch, **dadurch gekennzeichnet, dass** die Nadel (14; 114; 214; 314) beweglich in einer Kammer (401) der Einspritzvorrichtung (400) angebracht ist.

## Claims

1. Mixing apparatus (1; 101; 201; 301) for mixing at least two ingredients coming from at least a first reservoir (3; 103; 202A; 303) and a second reservoir (2; 102; 202B; 302) distinct from said first reservoir (3; 103; 202A; 303), said mixing apparatus (1; 101; 201; 301) comprising at least one first orifice (7; 107; 206A; 307) suitable for being connected to said first reservoir (3; 103; 202A; 303), and at least one second orifice (6; 106A, 106B; 206B, 206C; 306) distinct from said first orifice (7; 107; 206A; 307) and suitable for being connected to said second reservoir (2; 102; 202B; 302), and a motor-driven pump (15; 115; 215; 315), said mixing apparatus being **characterized in that** it includes a fluid-connection selector (9; 109; 209; 309) connected to said pump (15; 115; 215; 315) and including a network of channels (12; 112; 212; 312), said fluid-connection selector (9; 109; 209; 309) being provided with at least:
· a stationary portion (10; 110; 210; 310) including at least a portion of said network of channels (12; 112; 212; 312) opening out at least via said first orifice (7; 107; 206A; 307) and via said second orifice (6; 106A, 106B; 206B, 206C; 306);
· a moving portion (11, 111; 211; 311) including at least one other portion of said network of channels (12; 112; 212; 312) and mounted to move between a plurality of positions including at least:
· a first position in which said network of channels (12; 112; 212; 312) puts said at least one first orifice (7; 107; 206A; 307) and said at least one second orifice (6; 106A, 106B; 206B, 206C; 306) into communication with said pump (15; 115; 215; 315) so as to make it possible, by means of said pump (15; 115; 215; 315), to transfer at least one ingredient from said first orifice (7; 107; 206A; 307) towards said second orifice (6; 106A; 106B; 206B; 206C; 306) via said pump (15; 115; 215; 315); and
· a second position in which said network of channels (12; 112; 212; 312) puts said second orifice (6; 106A, 106B; 206B, 206C; 306) into communication with said pump (15; 115; 215; 315) so as to make it possible, by means of said pump (15; 115; 215; 315), to blend the resulting mixture by causing it to flow through said network of channels (12; 112; 212; 312).

2. Mixing apparatus (1; 301) according to claim 1, **characterized in that** said pump (15; 315) is at least suitable for being coupled to a pump reservoir.

3. Mixing apparatus (101; 201) according to any preceding claim, **characterized in that** it is provided with two second orifices (106A, 106B; 206B, 206C) suitable for being coupled to said second reservoir (102; 202B) so that, in said second position, said blending is obtained by said pump (115, 215) causing said mixture to flow between said second orifices (106A, 106B; 206B, 206C).

4. Mixing apparatus (1; 101; 201; 301) according to any preceding claim, **characterized in that** said network of channels (12; 112; 212; 312) of said fluid-connection selector (9; 109; 209; 309) includes at least one outlet channel (13; 113, 213; 313) for discharging said mixture from said apparatus (1; 101; 201; 301), **in that** said moving portion (11; 111; 211; 311) of said fluid-connection selector (9; 109; 209; 309) can take up a third position in which said network of channels (12; 112; 212; 312) puts said pump (15; 115; 215; 315) or said second orifice (6; 106A, 106B; 206B, 206C; 306) into communication with said outlet channel (13; 113; 213; 313) in order to discharge said mixture from said mixing apparatus (1; 101; 201; 301).

5. Mixing apparatus (201) according to any preceding claim, **characterized in that** it has at least one third orifice (206D) suitable for being connected to a third reservoir (202C) distinct from said first and second reservoirs (202B, 202A), **in that** said moving portion (211) of said fluid-connection selector (209) is suitable for being in a third position in which said network of channels (212) puts said second orifice (206B, 206C) and said third orifice (206D) into communication with each other in order to enable said mixture to be transferred from said second orifice (206B, 206C) towards said third orifice (206D).

6. Mixing apparatus (1; 101; 201; 301) according to any preceding claim, **characterized in that** at least one of said first orifice (s) (7; 107; 206A; 307), of said second orifice(s) (6; 106A, 106B; 206B, 206C; 306) and of said third orifice(s) (206D) is formed individually on an end-piece, and **in that**, when said mixing apparatus (1; 101; 201; 301) has two second orifices (106A, 106B; 206B, 206C), the end-pieces carrying said two second orifices have different lengths, making it possible to improve the flow of fluid through said second reservoir (2; 102; 202B; 302).

7. Mixing apparatus (1; 101; 201; 301) according to any preceding claim, **characterized in that** at least one of said first orifice (s) (7; 107; 206A; 307), of said second orifice(s) (6; 106A, 106B; 206B, 206C; 306) and of said third orifice(s) (206D) is formed individually on an end-piece, and **in that**, when said mixing apparatus (1; 101; 201; 301) has two second orifices (106A, 106B; 206B, 206C), said two second orifices have mutually different inside end section dimensions, making it possible to improve the flow of fluid through said second reservoir (2; 102; 202B; 302).

8. Mixing apparatus (1; 101; 201; 301) according to any preceding claim, **characterized in that** said fluid-connection selector (9; 109; 209; 309) is arranged to be coupled to a motor drive (18; 118; 218; 318) suitable for moving it between said first, second, and third positions.

9. Mixing apparatus (1; 101; 201; 301) according to at least claim 8, **characterized in that** it includes a disposable first assembly (4; 104; 204; 304) including said fluid-connection selector (9; 109; 209; 309), and a reusable second assembly (5; 105; 205; 305), including said motor drive (18; 118; 218; 318) of said pump (15; 115; 215; 315) and of said fluid-connection selector (9; 109; 209; 309), said disposable first assembly (4; 104; 204; 304) and said reusable second assembly (5; 105; 205; 305) being arranged to be connectable and detachable respectively one relative to the other.

10. mixing apparatus (1; 101; 201; 301) according to at least claim 9, **characterized in that** said disposable first assembly (4; 104; 204; 304) includes at least said first reservoir (3; 103; 202A; 303) containing a solvent and said second reservoir (2; 102; 202B; 302) containing a solid powder or a lyophilized substance.

11. Mixing apparatus (1; 101) according to any one of claims 1 to 10, **characterized in that** said moving portion (11, 111) of said fluid-connection selector (9; 109) is arranged to be movable in rotation relative to said stationary portion (10; 110).

12. Mixing apparatus (201; 301) according to any one of claims 1 to 9, **characterized in that** said moving portion (211, 311) of said fluid-connection selector (209; 309) is arranged to be movable in translation relative to said stationary portion (210; 310).

13. Medical-use miniaturized injection apparatus (400), **characterized in that** it includes mixing apparatus (1; 101; 201; 301) according to any preceding claim, and a needle (14, 114; 214; 314) connected to said fluid-connection selector (9; 109; 209; 309).

14. Injection apparatus (400) according to the preceding claim, **characterized in that** said needle (14; 114; 214; 314) is mounted to move in an enclosure (401) of said injection apparatus (400).
